(19) Europäisches Patentamt
European Patent Office
Office européen des brevets

(11) Veröffentlichungsnummer: **0 599 203 A1**

# (12) EUROPÄISCHE PATENTANMELDUNG

(21) Anmeldenummer: **93118595.3**

(22) Anmeldetag: **18.11.93**

(51) Int. Cl.[5]: **C07C 233/79**, C07C 311/20, C07C 237/06, C07C 255/24, A61K 31/16, A61K 31/18, C07C 233/41, C07C 311/07, C07C 233/62, C07D 333/38, A61K 31/38

(30) Priorität: **20.11.92 DE 4239151**

(43) Veröffentlichungstag der Anmeldung:
**01.06.94 Patentblatt 94/22**

(84) Benannte Vertragsstaaten:
**AT BE CH DE DK ES FR GB GR IE IT LI LU NL PT SE**

(71) Anmelder: **Dr. Karl Thomae GmbH**

**D-88397 Biberach(DE)**

(72) Erfinder: **Maier, Roland, Dr. Dipl.-Ing.Chem.**
**Bodelschwinghstrasse 39**
**D-88400 Biberach(DE)**
Erfinder: **Müller, Peter, Dr.Dipl. Chem.**
**Buchenweg 6**
**D-88441 Mittelbiberach(DE)**
Erfinder: **Wiotun, Eberhard, Dr.Dipl.-Ing.**
**Stecherweg 17**
**D-88400 Biberach(DE)**
Erfinder: **Hurnaus, Rudolf, Dr. Dipl.-Ing.**
**Silcherstrasse 19**
**D-88400 Biberach(DE)**
Erfinder: **Mark, Michael, Dr.**
**Schlehenhang 17**
**D-88400 Biberach(DE)**
Erfinder: **Eisele, Bernhard, Dr. Dipl.-Chem.**
**Beethovenstrasse 12**
**D-88400 Biberach(DE)**
Erfinder: **Budzinski, Ralph-Michael, Dr. Dipl.-Biologe**
**Thüringenstrasse 28**
**D-88400 Biberach(DE)**
Erfinder: **Hallermayer, Gerhard, Dr.Dipl.-Chem.**
**Grüner Weg 8**
**D-88437 Maselheim-Sulmingen(DE)**

(54) **N,n,-Disubstituierte Arylcycloalkylamine, deren Salze, diese Verbindungen enthaltende Arzneimittel und deren Verwendung sowie Verfahren zu iher Herstellung.**

(57) Die Erfindung betrifft N,N-disubstituierte Arylcycloalkylamine der allgemeinen Formel

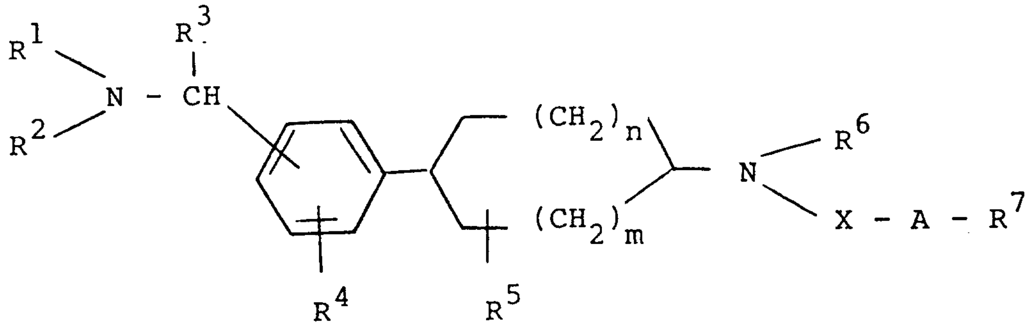

(I)

**EP 0 599 203 A1**

in der
n, m, A, X und $R^1$ bis $R^7$ wie im Anspruch 1 definiert sind deren Isomeren, Isomerengemische und deren Salze, welche wertvolle Eigenschaften aufweisen, insbesondere eine inhibitorische Wirkung auf die Cholesterolbiosynthese ausüben.

Die vorliegende Erfindung betrifft N,N-disubstituierte Arylcycloalkylamine, ihre Salze mit physiologisch verträglichen organischen und anorganischen Säuren, Verfahren zur Herstellung dieser Verbindungen und diese enthaltende Arzneimittel und deren Verwendung.

Die erfindungsgemäßen Verbindungen stellen Inhibitoren der Cholesterolbiosynthese dar, insbesondere Inhibitoren des Enzyms 2,3-Epoxisqualen-Lanosterol-Cyclase, eines Schlüsselenzyms der Cholesterolbiosynthese. Die erfindungsgemäßen Verbindungen sind geeignet zur Behandlung und Prophylaxe von Hyperlipidämien, Hypercholesterolämien und der Atherosklerose. Weitere mögliche Anwendungsgebiete ergeben sich für die Behandlung von hyperproliferativen Haut- und Gefäßerkrankungen, Tumoren, Gallensteinleiden sowie von Mykosen.

Verbindungen, die in die Cholesterolbiosynthese eingreifen, sind für die Behandlung einer Reihe von Krankheitsbildern von Bedeutung. Hier sind vor allem Hypercholesterolämien und Hyperlipidämien zu nennen, die Risikofaktoren für das Entstehen atherosklerotischer Gefäßveränderungen und ihrer Folgeerkrankungen wie beispielsweise koronare Herzkrankheit, cerebrale Ischämie, Claudicatio intermittens und Gangrän darstellen.

Die Bedeutung überhöhter Serum-Cholesterol-Spiegel als Hauptrisikofaktor für das Entstehen atherosklerotischer Gefäßveränderungen wird allgemein anerkannt. Umfangreiche klinische Studien haben zu der Erkenntnis geführt, daß durch Erniedrigung des Serumcholesterols das Risiko für koronare Herzerkrankungen verkleinert werden kann (Current Opinion in Lipidology 2(4), 234 [1991]). Da der größte Teil des Cholesterols im Organismus selbst synthetisiert und nur ein geringer Teil mit der Nahrung aufgenommen wird, stellt die Hemmung der Biosynthese einen besonders attraktiven Weg dar, den erhöhten Cholesterolspiegel zu senken.

Daneben kommen als weitere mögliche Anwendungsgebiete von Cholesterolbiosyntheseinhibitoren die Behandlung hyperproliferativer Haut- und Gefäßerkrankungen sowie von Tumorerkrankungen, die Behandlung und Prophylaxe von Gallensteinleiden sowie der Einsatz bei Mykosen in Betracht. Im letzteren Fall handelt es sich um einen Eingriff in die Ergosterolbiosynthese in Pilzorganismen, welche weitgehend analog der Cholesterolbiosynthese in Säugerzellen verläuft.

Die Cholesterol- bzw. die Ergosterolbiosynthese verläuft, ausgehend von Essigsäure, über eine größere Zahl von Reaktionsschritten. Dieser Vielstufenprozeß bietet eine Reihe von Eingriffsmöglichkeiten, von denen als Beispiele genannt seien:

Für die Inhibition des Enzyms 3-Hydroxy-3-methylglutaryl-Coenzym A (HMG-CoA)-Synthase werden β-Lactone und β-Lactame mit potentieller antihypercholesterolämischer Wirkung erwähnt (siehe J. Antibiotics 40, 1356 [1987], US-A-4,751,237, EP-A-0 462 667, US-A-4,983,597).

Inhibitoren des Enzyms HMG-CoA-Reduktase stellen 3,5-Dihydroxycarbonsäuren vom Statintyp und deren δ-Lactone dar, deren Vertreter Lovastatin, Simvastatin und Pravastatin in der Therapie von Hypercholesterolämien Verwendung finden. Weitere mögliche Anwendungsgebiete dieser Verbindungen sind Pilzinfektionen (US-A-4,375,475, EP-A-0 113 881, US-A-5,106,992), Hauterkrankungen (EP-A-0 369 263) sowie Gallensteinleiden und Tumorerkrankungen (US-A-5,106,992; Lancet 339, 1154-1156 [1992]). Eine weitere Therapiemöglichkeit stellt die Hemmung der Proliferation glatter Muskelzellen durch Lovastatin dar (Cardiovasc. Drugs. Ther. 5, Suppl. 3, 354 [1991]).

Inhibitoren des Enzyms Squalen-Synthetase sind z. B. Isoprenoid-(phosphinylmethyl)phosphonate, deren Eignung zur Behandlung von Hypercholesterolämien, Gallensteinleiden und Tumorerkrankungen beschrieben ist in EP-A-0 409 181 sowie J. Med. Chemistry 34, 1912 [1991], ferner die Squalestatine mit cholesterolsenkender und antimykotischer Wirkung (J. Antibotics 45, 639-647 [1992] und J. Biol. Chemistry 267, 11705-11708 [1992].

Als Inhibitoren des Enzyms Squalen-Epoxidase sind bekannt Allylamine wie Naftifin und Terbinafin, die als Mittel gegen Pilzerkrankungen Eingang in die Therapie gefunden haben, sowie das Allylamin NB-598 mit antihypercholesterolämischer Wirkung (J. Biol. Chemistry 265, 18075-18078, [1990]) und Fluorsqualen-Derivate mit hypocholesterolämischer Wirkung (US-A-5,011,859). Des weiteren sind Piperidine und Azadecaline mit potentieller hypocholesterolämischer und/oder antifungaler Wirkung beschrieben, deren Wirkmechanismus nicht eindeutig geklärt ist und welche Squalenepoxidase- und/oder 2,3-Epoxisqualen-Lanosterol-Cyclase-Inhibitoren darstellen (EP-A-0 420 116, EP-A-0 468 434, US-A-5,084,461 und EP-A-0 468 457).

Beispiele für Inhibitoren des Enzyms 2,3-Epoxisqualen-Lanosterol-Cyclase sind Diphenylderivate (EP-A-0 464 465), Aminoalkoxybenzol-Derivate (EP-A-0 410 359) sowie Piperidin-Derivate (J. Org. Chem. 57, 2794-2803, [1992]), die eine antifungale Wirkung besitzen. Des weiteren wird dieses Enzym in Säugetierzellen durch Decaline, Azadecaline und Indanderivate (WO 89/08450, J. Biol. Chemistry 254, 11258-11263 [1981], Biochem. Pharmacology 37, 1955-1964 [1988] und J 64 003 144), ferner durch 2-Aza-2,3-dihydrosqualen und 2,3-Epiminosqualen (Biochem. Pharmacology 34, 2765-2777 [1985]), durch Squalenoid-Epoxid-Vinylether (J. Chem. Soc. Perkin Trans. I, 1988, 461) und 29-Methyliden-2,3-oxidosqualen (J. Amer.

Chem. Soc. 113, 9673-9674 [1991]) inhibiert.

Schließlich sind als Inhibitoren des Enzyms Lanosterol-14α-Demethylase noch Steroidderivate mit potentieller antihyperlipämischer Wirkung zu nennen, die gleichzeitig das Enzym HMG-CoA-Reduktase beeinflussen (US-A-5,041,432, J. Biol. Chemistry 266, 20070-20078 [1991], US-A-5,034,548). Außerdem wird dieses Enzym durch die Antimykotika vom Azol-Typ inhibiert, welche N-substituierte Imidazole und Triazole darstellen. Zu dieser Klasse gehören beispielsweise die auf dem Markt befindlichen Antimykotika Ketoconazol und Fluconazol.

Die Verbindungen der nachfolgenden allgemeinen Formel I sind neu. Es wurde überraschenderweise gefunden, daß sie sehr wirksame Inhibitoren des Enzyms 2,3-Epoxisqualen-Lanosterol-Cyclase (Internationale Klassifizierung: EC5.4.99.7) darstellen.

Das Enzym 2,3-Epoxisqualen-Lanosterol-Cyclase katalysiert einen Schlüsselschritt der Cholesterol- bzw. Ergosterol-Biosynthese, nämlich die Umwandlung des 2,3-Epoxisqualens in das Lanosterol, die erste Verbindung mit Steroidstruktur in der Biosynthesekaskade. Inhibitoren dieses Enzyms lassen gegenüber Inhibitoren früherer Biosyntheseschritte, wie beispielsweise HMG-CoA-Synthase und HMG-CoA-Reduktase, den Vorteil der höheren Selektivität erwarten, da die Inhibierung dieser frühen Biosyntheseschritte zur Abnahme biosynthetisch gebildeter Mevalonsäure führt und dadurch auch die Biosynthese der mevalonsäureabhängigen Substanzen Dolichol, Ubichinon und Isopentenyl-t-RNA negativ beeinflussen kann (vgl. J. Biol. Chemistry 265, 18075-18078 [1990]).

Bei Inhibierung von Biosyntheseschritten nach der Umwandlung von 2,3-Epoxisqualen in Lanosterol besteht die Gefahr der Anhäufung von Intermediärprodukten mit Steroidstruktur im Organismus und der Auslösung dadurch bedingter toxischer Effekte. Dies ist beispielsweise für Triparanol, einem Desmosterol-Reduktase-Inhibitor, beschrieben. Diese Substanz mußte wegen Bildung von Katarakten, Ichthyosis und Alopecie vom Markt genommen werden (zitiert in J. Biol. Chemistry 265, 18075-18078 [1990]).

Wie bereits eingangs dargelegt sind Inhibitoren der 2,3-Epoxisqualen-Lanosterol-Cyclase vereinzelt in der Literatur beschrieben. Die Strukturen dieser Verbindungen sind jedoch völlig verschieden von der Struktur der erfindungsgemäßen Verbindungen der nachstehend genannten allgemeinen Formel I.

Die Erfindung betrifft die Bereitstellung von antihypercholesterolämischen Substanzen, die zur Behandlung und Prophylaxe der Atherosklerose geeignet sind und, im Vergleich zu bekannten Wirkstoffen, durch eine bessere antihypercholesterolämische Wirkung bei erhöhter Selektivität und damit erhöhter Sicherheit ausgezeichnet sind. Da die erfindungsgemäßen Verbindungen auf Grund ihrer hohen Wirksamkeit als Inhibitoren des Enzyms 2,3-Epoxisqualen-Lanosterol-Cyclase auch die Ergosterol-Biosynthese im Pilzorganismus inhibieren können, sind sie auch zur Behandlung von Mykosen geeignet.

Die N,N-disubstituierten Arylcycloalkylamine der vorliegenden Erfindung und ihre Salze besitzen die allgemeine Formel I. Die Verbindungen können gegebenenfalls auch in Form von Enantiomeren, Diastereomeren oder deren Gemischen vorliegen.

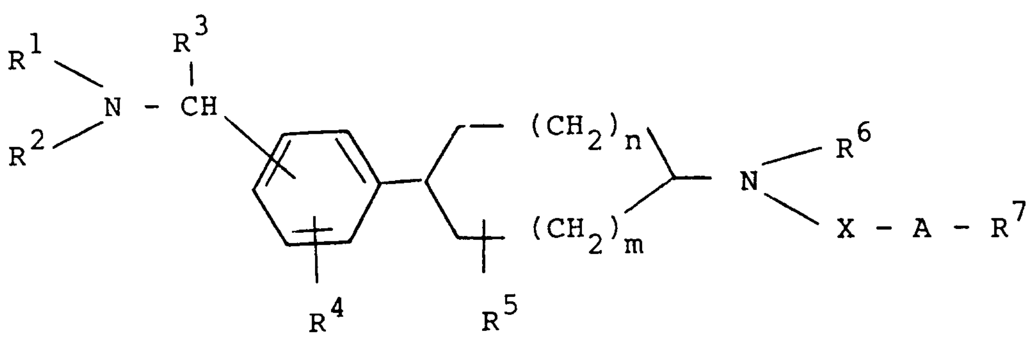

(I)

In der allgemeinen Formel I bedeuten

n die Zahlen 1 oder 2,

m die Zahlen 0 oder 1,

A eine Einfachbindung, eine geradkettige oder verzweigte Alkylengruppe mit 1 bis 17 Kohlenstoffatomen, eine Alkenylengruppe mit 2 bis 17 Kohlenstoffatomen oder eine Alkinylengruppe mit 2 bis 4 Kohlenstoffatomen,

X eine Carbonyl- oder Sulfonylgruppe,

$R^1$ eine geradkettige oder verzweigte Alkylgruppe mit 1 bis 5 Kohlenstoffatomen,

$R^2$ eine geradkettige oder verzweigte Alkylgruppe mit 1 bis 5 Kohlenstoffatomen, die durch eine oder zwei Hydroxygruppen, eine Alkoxy- oder durch eine Alkylcarbonyloxygruppe mit 1 bis 5 Kohlenstoffatomen im

Alkylteil, wobei der Alkylteil geradkettig oder verzweigt sein kann, durch eine Alkoxycarbonyloxygruppe, wobei die vorstehend erwähnten Substituenten nicht in Position 1 der Alkylgruppe gebunden sein können und zwei dieser Reste nicht an dasselbe Kohlenstoffatom gebunden sein können, sowie durch eine Aminocarbonyl-, Alkylaminocarbonyl-, Dialkylaminocarbonyl-, Cyano- oder Alkylcarbonylgruppe substituiert sein kann,
$R^3$, $R^4$ und $R^5$, die gleich oder verschieden sein können, jeweils ein Wasserstoffatom oder eine Alkylgruppe,
$R^6$ ein Wasserstoffatom, eine geradkettige oder verzweigte Alkylgruppe mit 1 bis 6 Kohlenstoffatomen, eine Cycloalkylgruppe mit 3 bis 6 Kohlenstoffatomen, eine Allyl-, Propargyl- oder eine gegebenenfalls durch ein Halogenatom substituierte Benzylgruppe,
$R^7$ ein Wasserstoffatom, eine Cycloalkylgruppe mit 3 bis 6 Kohlenstoffatomen, eine gegebenenfalls durch eine Alkylgruppe, durch ein oder zwei Halogenatome oder eine Trifluormethylgruppe substituierte Phenylgruppe, eine Naphthyl-, Tetrahydronaphthyl- oder eine gegebenenfalls durch ein Halogenatom oder eine Alkylgruppe substituierte Thienylgruppe,
wobei A keine Einfachbindung sein kann, wenn X die Sulfonylgruppe und $R^7$ ein Wasserstoffatom bedeuten, und
wobei, sofern nichts anderes erwähnt wurde, die vorstehend erwähnten Alkyl- und Alkoxyteile jeweils 1 bis 3 Kohlenstoffatome enthalten können und die vorstehend erwähnten Halogenatome jeweils ein Fluor-, Chlor- oder Bromatom bedeuten können.

Bevorzugt sind die Verbindungen der allgemeinen Formel I,
in der
n die Zahl 1,
m die Zahl 1,
A eine Einfachbindung, eine geradkettige oder verzweigte Alkylengruppe mit 1 bis 17 Kohlenstoffatomen oder eine Alkenylengruppe mit 2 bis 4 Kohlenstoffatomen,
X eine Carbonyl- oder Sulfonylgruppe,
$R^1$ eine geradkettige oder verzweigte Alkylgruppe mit 1 bis 5 Kohlenstoffatomen,
$R^2$ eine geradkettige oder verzweigte Alkylgruppe mit 1 bis 5 Kohlenstoffatomen, die durch eine oder zwei Hydroxygruppen, eine Alkoxy- oder durch eine Alkylcarbonyloxygruppe mit 1 bis 5 Kohlenstoffatomen im Alkylteil, wobei der Alkylteil geradkettig oder verzweigt sein kann, durch eine Alkoxycarbonyloxygruppe, wobei die vorstehend erwähnten Substituenten nicht in Position 1 der Alkylgruppe gebunden sein können und zwei dieser Reste nicht an dasselbe Kohlenstoffatom gebunden sein können, sowie durch eine Aminocarbonyl-, Cyano- oder Alkylcarbonylgruppe substituiert sein kann,
$R^3$ ein Wasserstoffatom oder eine Alkylgruppe,
$R^4$ und $R^5$, die gleich oder verschieden sein können, jeweils ein Wasserstoffatom oder eine Methylgruppe,
$R^6$ eine geradkettige oder verzweigte Alkylgruppe mit 1 bis 6 Kohlenstoffatomen, eine Cycloalkylgruppe mit 3 bis 6 Kohlenstoffatomen, eine Allyl-, Propargyl- oder eine gegebenenfalls durch ein Halogenatom substituierte Benzylgruppe,
$R^7$ ein Wasserstoffatom, eine Cycloalkylgruppe mit 3 bis 6 Kohlenstoffatomen oder eine gegebenenfalls durch 1 oder 2 Halogenatome oder eine Trifluormethylgruppe substituierte Phenylgruppe,
wobei A keine Einfachbindung sein kann, wenn X die Sulfonylgruppe und $R^7$ ein Wasserstoffatom bedeuten, und
wobei, sofern nichts anderes erwähnt wurde, die vorstehend erwähnten Alkyl- und Alkoxyteile jeweils 1 bis 3 Kohlenstoffatome enthalten können und die vorstehend erwähnten Halogenatome jeweils ein Fluor-, Chlor- oder Bromatom bedeuten können, deren Enantiomere, Diastereomere und deren Salze.

Besonders bevorzugt sind die Verbindungen der allgemeinen Formel I, in der
n die Zahl 1,
m die Zahl 1,
A eine Einfachbindung, eine geradkettige oder verzweigte Alkylengruppe mit 1 bis 17 Kohlenstoffatomen oder eine 2-Propenylengruppe,
X eine Carbonyl- oder Sulfonylgruppe,
$R^1$ eine geradkettige oder verzweigte Alkylgruppe mit 1 bis 5 Kohlenstoffatomen,
$R^2$ eine geradkettige oder verzweigte Alkylgruppe mit 1 bis 5 Kohlenstoffatomen, die durch eine oder zwei Hydroxygruppen, eine Alkoxy- oder durch eine Alkylcarbonyloxygruppe mit 1 bis 4 Kohlenstoffatomen im Alkylteil, wobei der Alkylteil geradkettig oder verzweigt sein kann, durch eine Alkoxycarbonyloxygruppe, wobei die vorstehend erwähnten Substituenten nicht in Position 1 der Alkylgruppe gebunden sein können und zwei dieser Reste nicht an dasselbe Kohlenstoffatom gebunden sein können, sowie durch eine Aminocarbonyl-, Cyano- oder Alkylcarbonylgruppe substituiert sein kann,

$R^3$ ein Wasserstoffatom oder eine Methylgruppe,
$R^4$ und $R^5$ jeweils ein Wasserstoffatom,
$R^6$ eine geradkettige oder verzweigte Alkylgruppe mit 1 bis 3 Kohlenstoffatomen, eine Cyclopropylgruppe, eine Allyl-, Propargyl- oder eine gegebenenfalls durch ein Fluoratom substituierte Benzylgruppe,
$R^7$ ein Wasserstoffatom, eine Cyclohexylgruppe, eine gegebenenfalls in 4-Stellung durch ein Chloratom oder eine Trifluormethylgruppe substituierte Phenylgruppe, die 3,4-Dichlorphenylgruppe, die 4-Chlor-3-methylphenylgruppe, die 5-Methyl-2-thienylgruppe, die 5-Chlor-2-thienylgruppe oder die 1,2,3,4-Tetrahydronaphthylgruppe,
wobei A keine Einfachbindung sein kann, wenn X die Sulfonylgruppe und $R^7$ ein Wasserstoffatom bedeuten, und
wobei, sofern nichts anderes erwähnt wurde, die vorstehend erwähnten Alkyl- und Alkoxyteile jeweils 1 bis 3 Kohlenstoffstoffatome enthalten können, deren Enantiomere, Diastereomere und deren Salze,
insbesondere jedoch die Verbindungen

(1) trans-N-(4-Chlorbenzolsulfonyl)-N-methyl-4-[4-(2-methoxyethyl)methylaminomethylphenyl]-cyclohexylamin
(2) trans-4-(4-Aminocarbonylmethyl-methylaminomethylphenyl)-N-(4-chlorbenzolsulfonyl)-N-methylcyclohexylamin
(3) trans-4-(4-Aminocarbonylmethyl-methylaminomethylphenyl)-N-(4-chlorbenzoyl)-N-methylcyclohexylamin
(4) trans-N-(4-Chlorbenzoyl)-N-methyl-4-[4-(3-hydroxypropyl)methylaminomethylphenyl]cyclohexylamin
(5) trans-4-[4-(3-Acetoxypropyl)methylaminomethylphenyl]-N-(4-chlorbenzoyl)-N-methylcyclohexylamin
(6) trans-N-(4-Chlorbenzoyl)-N-methyl-4-[4-(3-cyanopropyl)methylaminomethylphenyl]cyclohexylamin
(7) trans-4-[4-(1-Aminocarbonylethyl)methylaminomethylphenyl]-N-(4-chlorbenzoyl)-N-methylcyclohexylamin
(8) trans-N-(4-Chlorbenzoyl)-N-methyl-4-[4-(R-2,3-dihydroxy-2-methylpropyl)methylaminomethylphenyl]-cyclohexylamin
(9) trans-N-(4-Chlorbenzoyl)-N-methyl-4-[4-(2-hydroxyethyl)isopropylaminomethylphenyl]cyclohexylamin
(10) Cis-N-(4-Chlorbenzoyl)-N-methyl-4-[4-(3-hydroxypropyl)methylaminomethylphenyl]cyclohexylamin
(11) trans-4-[4-(3-Hydroxypropyl)methylaminomethylphenyl]-N-methyl-N-(4-trifluormethylbenzoyl)-cyclohexylamin
(12) trans-N-Cyclohexylcarbonyl-N-methyl-4-[4-(3-hydroxypropyl)methylaminomethylphenyl]-cyclohexylamin
(13) trans-N-(4-Chlor-3-methylbenzoyl)-N-methyl-4-[4-(3-hydroxypropyl)methylaminomethylphenyl]-cyclohexylamin
(14) trans-N-(3,4-Dichlorphenylacetyl)-N-methyl-4-[4-(3-hydroxypropyl)methylaminomethylphenyl]-cyclohexylamin
(15) trans-4-[4-(3-Hydroxypropyl)methylaminomethylphenyl]-N-methyl-N-(1,2,3,4-tetrahydronaphthalin-2-carbonyl)cyclohexylamin
(16) trans-N-(5-Chlorthienyl-2-carbonyl)-N-methyl-4-[4-(3-hydroxypropyl)methylaminomethylphenyl]-cyclohexylamin

deren Enantiomere, Diastereomere und deren Salze.

Herstellungsmethoden:

Die Verbindungen der allgemeinen Formel I lassen sich nach folgenden Methoden darstellen:
a) Durch Umsetzung von Verbindungen der allgemeinen Formel II,

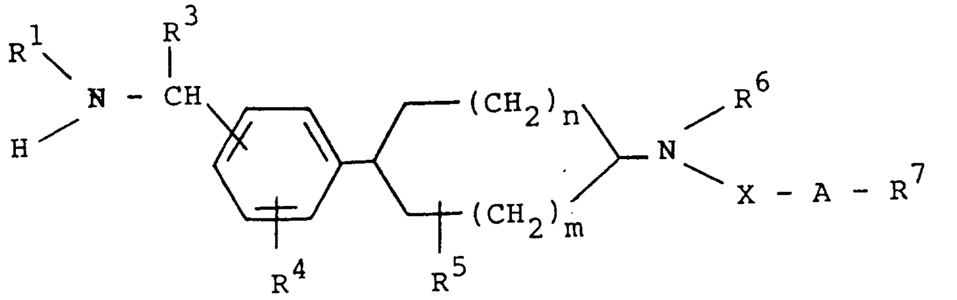 (II)

in der
n, m, A, X, $R^1$ und $R^3$ bis $R^7$ wie eingangs erwähnt definiert sind, mit einer Verbindung der allgemeinen Formel III,

$R^2$ - Y (III)

in der
$R^2$ wie eingangs erwähnt definiert ist und Y eine reaktive Austrittsgruppe wie ein Halogenatom, beispielsweise ein Chlor-, Brom- oder Iodatom, oder eine Sulfonyloxygruppe bedeutet.

Die Umsetzung wird zweckmäßigerweise in einem geeigneten Lösungsmittel wie Ethanol, tert.Butanol, Tetrahydrofuran, Dimethylformamid oder Dimethylsulfoxid oder in einem Gemisch der vorstehend erwähnten Lösungsmittel, gegebenenfalls in Gegenwart einer Base wie beispielsweise Kaliumcarbonat, Natriumcarbonat oder eines schwer alkylierbaren tert.Amins, wie z. B. N-Ethyldiisoproylamin, bei Temperaturen zwischen 0 °C und 100 °C, vorzugsweise jedoch bei einer Temperatur zwischen 0 und 40 °C, durchgeführt.

b) Verbindungen der allgemeinen Formel I, in der $R^2$ eine Gruppe der allgemeinen Formel IV,

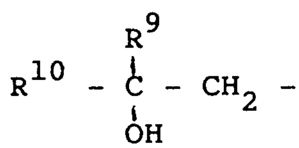
(IV)

in der
$R^9$ ein Wasserstoffatom oder eine Methylgruppe und $R^{10}$ eine Methyl- oder Hydroxymethylgruppe bedeutet:
Durch Umsetzung einer Verbindung der allgemeinen Formel II, in der n, m, A, X, $R^1$ und $R^3$ bis $R^7$ wie eingangs erwähnt definiert sind, mit einem Epoxid der allgemeinen Formel V,

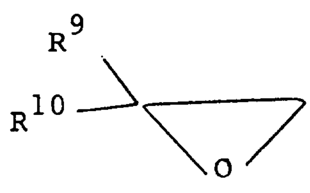
(V)

in der
$R^9$ und $R^{10}$ wie vorstehend erwähnt definiert sind.

Die Umsetzung wird zweckmäßigerweise in einem geeigneten Lösungsmittel wie Ethanol, tert.Butanol, Tetrahydrofuran, Dimethylformamid oder Dimethylsulfoxid oder in einem Gemisch der vorstehend erwähnten Lösungsmittel bei Temperaturen zwischen 0 °C und 100 °C, vorzugsweise jedoch bei einer Temperatur zwischen 0 und 40 °C, durchgeführt.

c) Durch Umsetzung von Verbindungen der allgemeinen Formel VI,

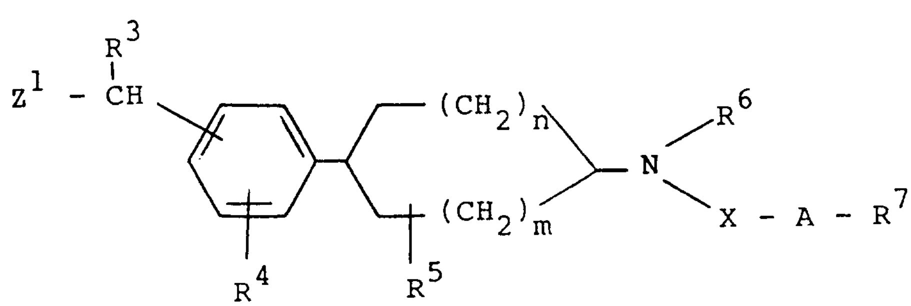
(VI)

in der

n, m, A, X und $R^3$ bis $R^7$ wie eingangs erwähnt definiert sind, und $Z^1$ eine reaktive Austrittsgruppe wie ein Halogenatom, beispielsweise ein Chlor- oder Bromatom, oder eine Sulfonyloxygruppe bedeutet, mit einem Amin der allgemeinen Formel VII,

$R^1R^2NH$ (VII)

in der
$R^1$ und $R^2$ wie eingangs erwähnt definiert sind.

Die Umsetzung wird zweckmäßigerweise in einem geeigneten Lösungsmittel wie Ethanol, tert.Butanol, Tetrahydrofuran, Dimethylformamid oder Dimethylsulfoxid oder in einem Gemisch der vorstehend erwähnten Lösungsmittel, gegebenenfalls in Gegenwart einer Base wie beispielsweise Kaliumcarbonat, Natriumcarbonat oder eines schwer alkylierbaren tert.Amins, wie z. B. N-Ethyldiisoproylamin, bei Temperaturen zwischen 0 °C und 100 °C, vorzugsweise jedoch bei einer Temperatur zwischen 0 und 40 °C, durchgeführt.

d) Durch Umsetzung von Verbindungen der allgemeinen Formel VIII,

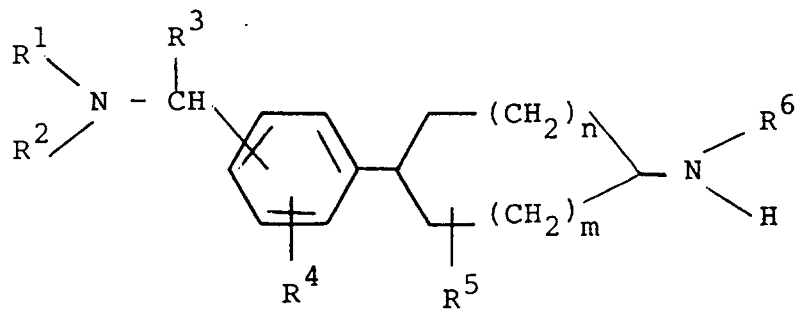

(VIII)

in der
n, m und $R^1$ bis $R^6$ die eingangs erwähnten Bedeutungen besitzen, mit einem Alkylierungsmittel der allgemeinen Formel IX,

$R^7$ - A - X - $Z^2$ (IX)

in der
A, X und $R^7$ die eingangs erwähnten Bedeutungen besitzen und $Z^2$ eine reaktive Austrittsgruppe wie z. B. ein Halogenatom, vorzugsweise ein Chloratom, oder die Imidazolidgruppe bedeutet.

Bedeutet $Z^2$ ein Halogenatom, werden die Umsetzungen in einem geeigneten inerten Lösungsmittel wie Diethylether, Toluol, Methylenchlorid und dergleichen, vorzugsweise bei Temperaturen zwischen -50 °C und 50 °C und in Gegenwart eines halogenwasserstoffbindenden Mittels, z. B. eines tertiären Amins, Natriumcarbonat oder Calciumcarbonat, durchgeführt. Dabei können nicht nur die freien Amine der allgemeinen Formel VIII eingesetzt werden, sondern auch deren Salze, aus denen in situ die Amine durch geeignete Basen, z. B. tertiäre organische Amine, freigesetzt werden können.

Bedeutet $Z^2$ den Imidazolidrest, werden die Umsetzungen vorzugsweise in einem inerten Lösungsmittel wie Xylol oder Tetrahydrofuran bei Temperaturen zwischen Raumtemperatur und der Siedetemperatur der Reaktionsmischung durchgeführt.

Besitzt eine Verbindung der allgemeinen Formel VIII eine oder zwei Hydroxygruppen, kann die Umsetzung so abgewandelt werden, daß zwei oder drei Äquivalente der Verbindung der allgemeinen Formel IX verwendet werden und nach beendeter Umsetzung die aus den Hydroxygruppen gebildeten Estergruppen wieder verseift werden.

Die gegebenenfalls anschließende Verseifung einer so gebildeten Estergruppe erfolgt vorzugsweise durch alkalische Hydrolyse in einem wässrigen Lösungsmittel, z.B. in Wasser, Isopropanol/Wasser, Tetrahydrofuran/Wasser oder Dioxan/Wasser, beispielsweise in Gegenwart einer Alkalibase wie Natriumhydroxid oder Kaliumhydroxid bei Temperaturen zwischen 0 und 100 °C.

Erhält man erfindungsgemäß eine Verbindung der allgemeinen Formel I, in der $R^2$ eine durch eine Hydroxygruppe substituierte Alkylgruppe darstellt, so kann diese durch Umsetzung mit einem geeigneten Acylierungsmittel in eine Verbindung der allgemeinen Formel I, in der $R^2$ eine durch eine Alkylcarbonyloxy- oder Alkoxycarbonyloxygruppe substituierte Alkylgruppe darstellt, übergeführt werden oder

mittels Oxidation in eine Verbindung der allgemeinen Formel I, in der $R^2$ eine Alkylcarbonylmethylgruppe darstellt, übergeführt werden.

Die nachträgliche Acylierung wird zweckmäßigerweise in einem Lösungsmittel oder Lösungsmittelgemisch Methylenchlorid, Chloroform, Äther, Tetrahydrofuran, Dioxan oder Dimethylformamid, mit Carbonsäureanhydriden wie Essigsäureanhydrid, mit Carbonsäurehalogeniden wie Acetylchlorid oder Chlorkohlensäurealkylestern gegebenenfalls in Gegenwart einer anorganischen oder tertiären organischen Base, wie Kaliumcarbonat, Triethylamin, N-Ethyldiisopropylamin oder Pyridin, wobei die drei letzteren gleichzeitig auch als Lösungsmittel dienen können, bei Temperaturen zwischen -25 und 100 °C, vorzugsweise jedoch bei Temperaturen zwischen -10 und 80 °C, durchgeführt.

Die nachträgliche Oxidation erfolgt zweckmäßigerweise in einem geeigneten Lösungsmittel oder Lösungsmittelgemisch, z. B. in Aceton, Pyridin, Wasser/Pyridin, Eisessig, Dichlormethan, Chloroform, Benzol oder Toluol bei Temperaturen zwischen -20 und 100 °C. Als Oxidationsmittel kommen z. B. Dimethylsulfoxid in Kombination mit N,N-Dicyclohexylcarbodiimid und Trifluoressigsäure (Oxidation nach Pfitzner-Moffatt), Chromsäure in Eisessig oder in Aceton, Mangandioxid in Chloroform oder Kaliumpermanganat in Eisessig, Pyridin oder in Aceton in Betracht.

Die nach den vorstehenden Verfahren hergestellten Verbindungen der allgemeinen Formel I lassen sich nach bekannten Methoden, z. B. Kristallisation, Destillation oder Chromatographie reinigen und isolieren.

Desweiteren können die erhaltenen Verbindungen der allgemeinen Formel I gewünschtenfalls in ihre Säureadditionssalze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Phosphorsäure, Fumarsäure, Bernsteinsäure, Milchsäure, Zitronensäure, Weinsäure oder Maleinsäure in Betracht.

In den erfindungsgemäßen Verbindungen der Formel I können der an den Cycloalkylring gebundene Arylrest sowie das Stickstoffatom entweder äquatoriale oder axiale Anordnung einnehmen. Die Erfindung umfaßt sowohl die reinen Isomeren als auch die Gemische der verschiedenen Isomeren.

Ausgangsmaterialien:

Die Ausgangsverbindungen der allgemeinen Formel II lassen sich aus Verbindungen der allgemeinen Formel X,

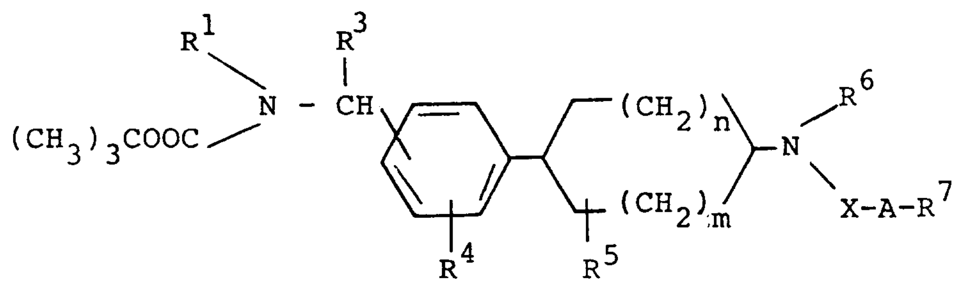

(X)

in der

n, m, A, X, $R^1$ und $R^3$ bis $R^7$ wie eingangs erwähnt definiert sind, durch Abspaltung des tert.Butoxycarbonylrestes darstellen.

Die Verbindungen der allgemeinen Formel X lassen sich ausgehend von Ketonen der allgemeinen Formel XI,

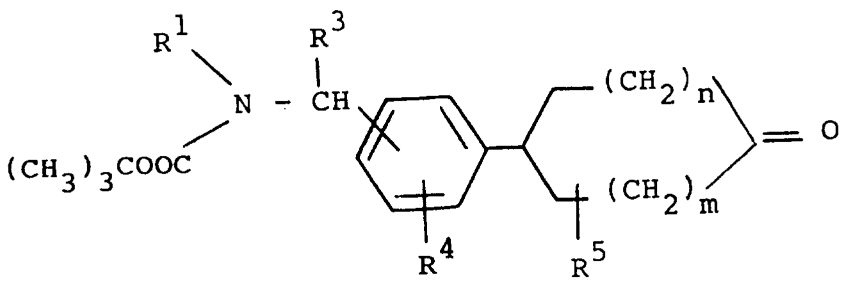

(XI)

in der
n, m, $R^1$ und $R^3$ bis $R^5$ wie eingangs erwähnt definiert sind, darstellen, indem diese zunächst mit Aminen der Formel $R^6NH_2$, in der $R^6$ wie eingangs erwähnt definiert ist, reduktiv aminiert werden und anschließend mit einem Acylierungsmittel der allgemeinen Formel IX,

$R^7$ - A - X - $Z^2$ (IX)

in der
A, X, $R^7$ und $Z^2$ wie vorstehend erwähnt definiert sind, umgesetzt werden.

Die Ketone der allgemeinen Formel XI lassen sich ausgehend von Verbindungen der Formel XII,

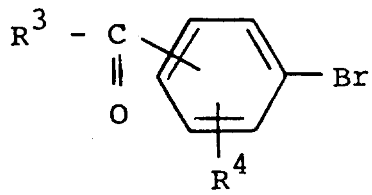

(XII)

in der
$R^3$ und $R^4$ wie eingangs erwähnt definiert sind, durch reduktive Aminierung mit Aminen der Formel $R^1NH_2$, in der $R^1$ wie eingangs erwähnt definiert ist, und anschließender Umsetzung der Aminogruppe mit Trimethylchlorsilan, Austausch des Bromatoms durch Lithium, Umsetzung der so erhaltenen lithiumorganischen Verbindung mit Verbindungen der allgemeinen Formel XIII,

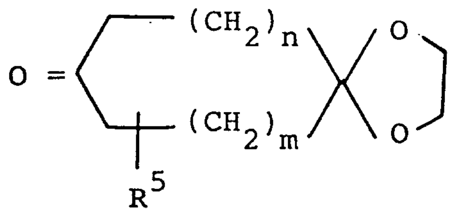

(XIII)

in der
n, m und $R^5$ wie eingangs erwähnt definiert sind, gefolgt von Wasserabspaltung, Einführung des tert.Butoxycarbonylrestes in die Aminogruppe, katalytischer Hydrierung der olefinischen Doppelbindung,beispielsweise unter Verwendung von Palladium/Bariumsulfat als Hydrierkatalysator, und abschließender Hydrolyse des Ethylenketals.

Ausgangsverbindungen der allgemeinen Formel VI, in der $R^3$ und $R^4$ jeweils ein Wasserstoffatom darstellen, n, m, $R^5$ und X wie eingangs erwähnt definiert sind, $R^6$, $R^7$ und A die eingangs erwähnten Bedeutungen mit Ausnahme halogenwasserstoffempfindlicher Reste, wie der Allyl-, Progargyl-, Cyclopropyl-, Alkenylen- oder Alkinylengruppe, besitzen, die Gruppierung $Z^1$-CH($R^3$)- in 4-Stellung zum Cycloalkylrest steht und $Z^1$ ein Chloratom darstellt, lassen sich durch Chlormethylierung von Verbindungen der allgemeinen Formel XIV,

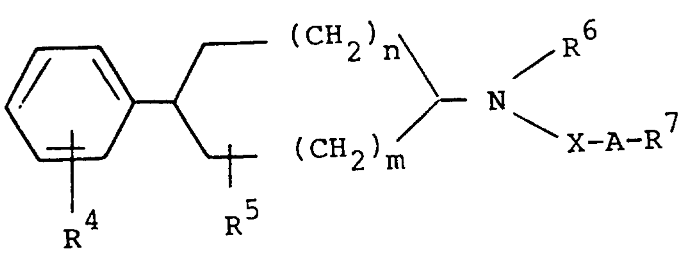

(XIV)

in der
$R^4$ ein Wasserstoffatom darstellt, n, m, $R^5$ und X wie eingangs erwähnt definiert sind und $R^6$, $R^7$ und A die

eingangs erwähnten Bedeutungen mit Ausnahme der oben genannten halogenwasserstoffempfindlichen Reste besitzen, erhalten.

Eine weitere Darstellungsmethode für Verbindungen der allgemeinen Formel VI, in der n, m, A, X und $R^3$ bis $R^7$ wie eingangs erwähnt definiert sind und $Z^1$ ein Bromatom oder eine Sulfonyloxygruppe bedeutet, besteht darin, eine Verbindung der allgemeinen Formel XV,

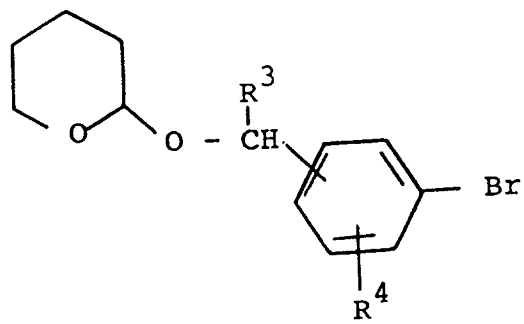

(XV)

in der
$R^3$ und $R^4$ wie eingangs erwähnt definiert sind, analog einer wie vorstehend für Verbindungen der Formel X beschriebenen Reaktionssequenz in Verbindungen der allgemeinen Formel XVI,

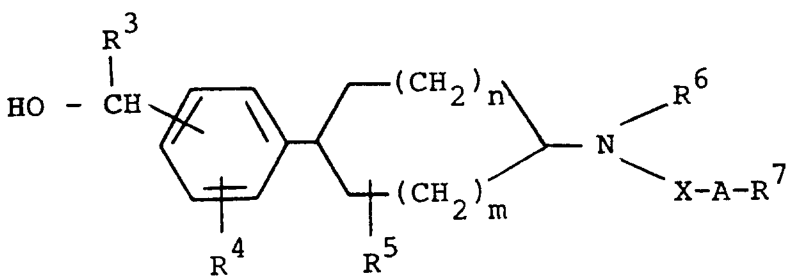

(XVI)

in der
n, m, A, X und $R^3$ bis $R^7$ wie eingangs erwähnt definiert sind, zu überführen und anschließend die Hydroxygruppe in die Sulfonyloxygruppe zu überführen oder durch ein Bromatom zu substituieren.

Die Ausgangsverbindungen der allgemeinen Formel VIII lassen sich dadurch herstellen, daß zunächst Verbindungen der allgemeinen Formel XVI, in der n, m und $R^3$ bis $R^6$ wie eingangs erwähnt definiert sind und der Rest $-X-A-R^7$ durch eine tert.Butoxycarbonylgruppe ersetzt ist, hergestellt werden und diese über die entsprechenden Sulfonyloxyverbindungen in Verbindungen der allgemeinen Formel XVII,

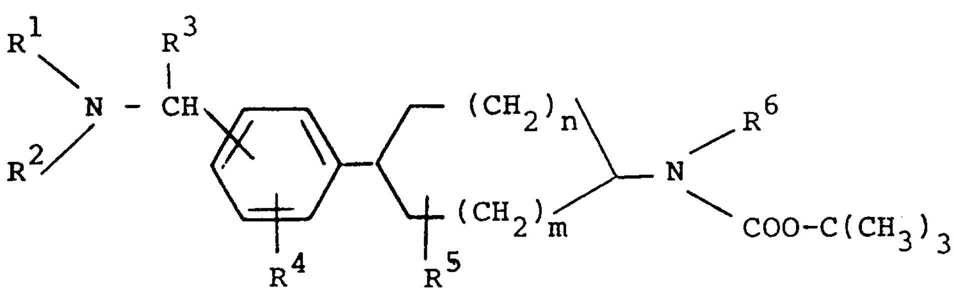

(XVII)

in der
n, m und $R^1$ bis $R^6$ wie eingangs erwähnt definiert sind, übergeführt werden, gefolgt von der Abspaltung des tert.Butoxycarbonylrestes.

Die Ausgangsverbindungen der allgemeinen Formel XIV lassen sich erhalten, indem zunächst eine Verbindung der Formel XVIII,

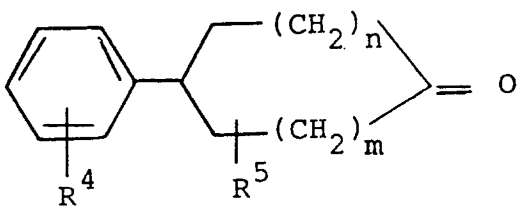

(XVIII)

in der
n, m, $R^4$ und $R^5$ wie eingangs erwähnt definiert sind, mit einem Amin der Formel $R^6NH_2$, in der $R^6$ wie eingangs erwähnt definiert ist, reduktiv aminiert wird und die so erhaltene Verbindung anschließend mit einem Acylierungsmittel der allgemeinen Formel IX,

$R^7$ - A - X -$Z^2$ (IX)

in der
A, X, $R^7$ und $Z^2$ wie vorstehend erwähnt definiert sind, umgesetzt wird.
Alternativ können zunächst Verbindungen der allgemeinen Formel XIV hergestellt werden, in der n, m, A, X, $R^4$, $R^5$ und $R^7$ die eingangs erwähnten Bedeutungen besitzen und $R^6$ ein Wasserstoffatom bedeutet, und diese anschließend mit einem Alkylierungsmittel der Formel $R^6$-Y, in der $R^6$ die vorstehend erwähnten Bedeutungen mit Ausnahme der eines Wasserstoffatoms besitzt und Y eine reaktive Austrittsgruppe wie ein Halogenatom, beispielsweise ein Chlor-, Brom- oder Iodatom, oder eine Sulfonyloxygruppe bedeutet, umzusetzen.

Die Verbindungen der allgemeinen Formel I besitzen interessante biologische Eigenschaften. Sie stellen Inhibitoren der Cholesterolbiosynthese, insbesondere Inhibitoren des Enzyms 2,3-Epoxisqualen-Lanosterol-Cyclase dar. Aufgrund ihrer biologischen Eigenschaften sind sie besonders geeignet zur Behandlung und Prophylaxe von Hyperlipidämien, insbesondere der Hypercholesterolämie, der Hyperlipoproteinämie und der Hypertriglyceridämie und den daraus resultierenden atherosklerotischen Gefäßveränderungen mit ihren Folgeerkrankungen wie koronare Herzkrankheit, cerebrale Ischämie, Claudicatio intermittens, Gangrän und andere.

Zur Behandlung dieser Erkrankungen können die Verbindungen der allgemeinen Formel I dabei entweder alleine zur Monotherapie eingesetzt werden oder in Kombination mit anderen cholesterol- oder lipidsenkenden Substanzen zur Anwendung gelangen, wobei die Verbindungen vorzugsweise als orale Formulierung, gegebenenfalls auch in Form von Suppositorien als rektale Formulierung verabreicht werden können. Als Kombinationspartner kommen dabei beispielsweise in Frage:

- gallensäurebindende Harze wie z. B. Cholestyramin, Cholestipol und andere,
- Verbindungen, die die Cholesterolresorption hemmen, wie z. B. Sitosterol und Neomycin,
- Verbindungen, die in die Cholesterolbiosynthese eingreifen, wie z. B. HMG-CoA-Reduktaseinhibitoren wie Lovastatin, Simvastatin, Pravastatin und andere,
- Squalen-Epoxidaseinhibitoren wie beispielsweise NB 598 und analoge Verbindungen sowie
- Squalen-Synthetaseinhibitoren wie beispielsweise Vertreter der Klasse der Isoprenoid-(phosphinylmethyl)phosphonate und Squalestatin.

Als weitere mögliche Kombinationspartner sind noch zu erwähnen die Klasse der Fibrate, wie Clofibrat, Bezafibrat, Gemfibrozil und andere, Nikotinsäure, ihre Derivate und Analoge wie beispielsweise Acipimox sowie Probucol.

Desweiteren sind die Verbindungen der allgemeinen Formel I geeignet zur Behandlung von Erkrankungen, die mit überhöhter Zellproliferation im Zusammenhang stehen. Cholesterol ist ein essentieller Zellbestandteil und muß für die Zellproliferation, d. h. Zellteilung, in ausreichender Menge vorhanden sein. Die Inhibierung der Zellproliferation durch Inhibierung der Cholesterolbiosynthese ist am Beispiel der glatten Muskelzellen mit dem HMG-CoA-Reduktaseinhibitor des Statintyps Lovastatin, wie eingangs erwähnt, beschrieben.

Als Beispiele für Erkrankungen, die mit überhöhter Zellproliferation zusammenhängen sind zunächst Tumorerkrankungen zu nennen. In Zellkultur- und in-vivo-Experimenten wurde gezeigt, daß die Senkung des Serumcholesterols oder der Eingriff in die Cholesterolbiosynthese durch HMG-CoA-Reduktaseinhibitoren das Tumorwachstum vermindert (Lancet 339, 1154-1156 [1992]). Die erfindungsgemäßen Verbindungen der Formel I sind deshalb aufgrund ihrer cholesterolbiosyntheseinhibitorischen Wirkung potentiell für die Behandlung von Tumorerkrankungen geeignet. Sie können dabei alleine oder zur Unterstützung bekannter

Therapieprinzipien Verwendung finden.

Als weitere Beispiele sind hyperproliferative Hauterkrankungen wie beispielsweise Psoriasis, Basalzellkarzinome, Plattenepithelkarzinome, Keratosis und Keratinisierungsstörungen zu nennen. Der hier verwendete Ausdruck "Psoriasis" bezeichnet eine hyperproliferativ-entzündliche Hauterkrankung, die den Regulierungsmechanismus der Haut verändert. Insbesondere werden Läsionen gebildet, die primäre und sekundäre Veränderungen der Proliferation in der Epidermis, entzündliche Reaktionen der Haut und die Expression regulatorischer Moleküle wie Lymphokine und Entzündungsfaktoren beinhalten. Psoriatische Haut ist morphologisch durch einen verstärkten Umsatz von Epidermiszellen, verdickte Epidermis, abnormale Keratinisierung entzündlicher Zellinfiltrate in die Dermisschicht und polymorphonucleäre Leukozyteninfiltration in die Epidermis, die eine Zunahme des Basalzellzyklus bedingt, gekennzeichnet. Zusätzlich sind hyperkeratotische und parakeratotische Zellen anwesend.

Der Ausdruck "Keratosis", "Basalzellkarzinome", "Plattenepithelkarzinome" und "Keratinisierungsstörungen" bezieht sich auf hyperproliferative Hauterkrankungen, bei denen der Regulierungsmechanismus für die Proliferation und Differenzierung der Hautzellen unterbrochen ist.

Die Verbindungen der Formel I sind wirksam als Antagonisten der Hauthyperproliferation, d. h. als Mittel, die die Hyperproliferation menschlicher Keratinozyten hemmen. Infolgedessen sind die Verbindungen als Mittel zur Behandlung hyperproliferativer Hauterkrankungen wie Psoriasis, Basalzellkarzinomen, Keratinisierungsstörungen und Keratosis geeignet.

Zur Behandlung dieser Krankheiten können die Verbindungen der Formel I entweder oral oder topisch appliziert werden, wobei sie entweder alleine in Form der Monotherapie oder in Kombination mit bekannten Wirkstoffen eingesetzt werden können.

Des weiteren zu nennen sind durch chirurgische Maßnahmen wie PTCA (perkutane transluminale coronare Angioplastie) oder Bypass-Operationen ausgelöste hyperproliferative Gefäßerkrankungen wie Stenosen und Gefäßverschlüsse, die auf der Proliferation glatter Muskelzellen beruhen. Wie eingangs erwähnt läßt sich diese Zellproliferation bekanntlich durch HMG-CoA-Reduktaseinhibitoren vom Statintyp, wie Lovastatin, unterdrücken. Aufgrund ihrer inhibitorischen Wirkung auf die Cholesterolbiosynthese sind auch die Verbindungen der allgemeinen Formel I geeignet zur Behandlung und Prophylaxe dieser Erkrankungen, wobei sie entweder alleine oder in Kombination mit bekannten Wirkstoffen, wie z. B. intravenös appliziertes Heparin, vorzugsweise in oraler Applikation Verwendung finden können.

Eine weitere Einsatzmöglichkeit der erfindungsgemäßen Verbindungen der allgemeinen Formel I ist die Prophylaxe und Behandlung von Gallensteinleiden. Die Gallensteinbildung wird dadurch ausgelöst, daß die Cholesterolkonzentration in der Galle die maximale Löslichkeit des Cholesterols in der Gallenflüssigkeit überschreitet, wodurch es zur Ausfällung des Cholesterols in Form von Gallensteinen kommt. Lipidsenker aus der Klasse der Fibrate führen zu einer erhöhten Ausscheidung von Neutralsteroiden über die Galle und erhöhen die Neigung zur Gallensteinbildung.

Im Gegensatz dazu führen Cholesterolbiosynthesehemmer wie Lovastatin oder Pravastatin zu keiner erhöhten Gallensteinbildung, sondern können im Gegenteil eine Reduktion der Cholesterolkonzentration in der Galle bewirken und damit den sogenannten lithogenen Index, ein Maß für die Wahrscheinlichkeit der Gallensteinbildung, vermindern. Dies ist beschrieben in Gut 31, 348-350 [1990] sowie in Z. Gastroenterol. 29, 242-245 [1991].

Darüber hinaus ist in Gastroenterology 102, No. 4, Pt. 2, A 319 [1992] die Wirksamkeit von Lovastatin bei der Auflösung von Gallensteinen, insbesondere in Kombination mit Ursodeoxycholsäure beschrieben. Aufgrund ihrer Wirkungsweise sind die Verbindungen der allgemeinen Formel I deshalb auch für die Prophylaxe und Behandlung von Gallensteinleiden von Bedeutung. Sie können dabei entweder allein oder in Kombination mit bekannten Therapien wie beispielsweise der Behandlung mit Ursodeoxycholsäure oder der Schockwellenlithotripsie vorzugsweise in oraler Applikation Verwendung finden.

Schließlich sind die Verbindungen der allgemeinen Formel I geeignet zur Therapie von Infektionen durch pathogene Pilze wie z. B. Candida albicans, Aspergillus niger, Trichophyton mentagrophytes, Penicillium sp., Cladosporium sp. und andere. Wie bereits eingangs erwähnt ist das Endprodukt der Sterolbiosynthese im Pilzorganismus nicht Cholesterol, sondern das für die Integrität und Funktion der Pilzzellmembranen essentielle Ergosterol. Die Inhibierung der Ergosterolbiosynthese führt deshalb zu Wachstumsstörungen und gegebenenfalls zur Abtötung der Pilzorganismen.

Zur Behandlung von Mykosen können die Verbindungen der allgemeinen Formel I entweder oral oder topisch appliziert werden. Dabei können sie entweder alleine oder in Kombination mit bekannten antimykotischen Wirkstoffen eingesetzt werden, insbesondere mit solchen, die in andere Stufen der Sterolbiosynthese eingreifen, wie beispielsweise den Squalen-Epoxidasehemmern Terbinafin und Naftifin oder den Lanosterol-14$\alpha$-Demethylaseinhibitoren vom Azol-Typ wie beispielsweise Ketoconazol und Fluconazol.

Eine weitere Verwendungsmöglichkeit der Verbindungen der allgemeinen Formel I betrifft die Anwendung in der Geflügelhaltung. Die Senkung des Cholesterolgehaltes von Eiern durch Verabreichung des HMG-CoA-Reduktaseinhibitors Lovastatin an Legehennen ist beschrieben (FASEB Journal 4, A 533, Abstracts 1543 [1990]). Die Erzeugung cholesterolarmer Eier ist von Interesse, da die Cholesterolbelastung des Körpers durch Eier mit reduziertem Cholesterolgehalt ohne eine Änderung der Ernährungsgewohnheiten vermindert werden kann. Aufgrund ihrer inhibitorischen Wirkung auf die Cholesterolbiosynthese können die Verbindungen der allgemeinen Formel I auch in der Geflügelzucht zur Erzeugung cholesterolarmer Eier Verwendung finden, wobei die Substanzen vorzugsweise als Zusatz zum Futter verabreicht werden.

Die biologische Wirkung von Verbindungen der allgemeinen Formel I wurden nach folgenden Methoden bestimmt:

I. Messung der Hemmung des $^{14}C$-Acetat-Einbaus in die mit Digitonin fällbaren Steroide:

Methode:

Humane Hepatoma-Zellen (HEP-G2) werden nach 3-tägiger Anzucht für 16 Stunden in cholesterolfreiem Medium stimuliert. Die zu testenden Substanzen (gelöst in Dimethylsulfoxid, Endkonzentration 0,1%) werden während dieser Stimulationsphase zugesetzt. Anschließend wird nach Zugabe von 200 µMol 1 2-$^{14}C$-Acetat für weitere zwei Stunden bei 37 °C im Brutschrank weiterinkubiert.

Nach Ablösung der Zellen und Verseifen der Sterolester wird nach Extraktion Digitonin zugesetzt und die ausgefällten Sterole isoliert. Das in die digitoninfällbaren Sterole eingebaute $^{14}C$-Acetat wird durch Szintillationsmessung bestimmt.

Die Untersuchung der Hemmwirkung wurde bei Testkonzentrationen von $10^{-7}$ Mol/l und $10^{-8}$ Mol/l durchgeführt. Es wurde gefunden, daß beispielsweise die folgenden Verbindungen A bis Q der allgemeinen Formel I bei diesen Testkonzentrationen eine gute Hemmwirkung zeigen, z. B. bei einer Testkonzentration von $10^{-7}$ Mol/l eine Hemmwirkung von mindestens 50%:

A = trans-N-(4-Chlorbenzolsulfonyl)-N-methyl-4-[4-(2-methoxyethyl)methylaminomethylphenyl]-cyclohexylamin

B = trans-4-(4-Aminocarbonylmethyl-methylaminomethylphenyl)-N-(4-chlorbenzolsulfonyl)-N-methylcyclohexylamin

C = trans-4-(4-Aminocarbonylmethyl-methylaminomethylphenyl)-N-(4-chlorbenzoyl)-N-methylcyclohexylamin

D = trans-N-(4-Chlorbenzoyl)-N-methyl-4-[4-(3-hydroxypropyl)methylaminomethylphenyl]cyclohexylamin

E = trans-4-[4-(3-Acetoxypropyl)methylaminomethylphenyl]-N-(4-chlorbenzoyl)-N-methylcyclohexylamin

F = trans-N-(4-Chlorbenzoyl)-N-methyl-4-[4-(3-cyanopropyl)methylaminomethylphenyl]cyclohexylamin

G = trans-4-[4-(1-Aminocarbonylethyl)methylaminomethylphenyl]-N-(4-chlorbenzoyl)-N-methylcyclohexylamin

H = trans-N-(4-Chlorbenzoyl)-N-methyl-4-[4-(R-2,3-dihydroxy-2-methylpropyl)-methylaminomethylphenyl]cyclohexylamin

I = trans-N-(4-Chlorbenzoyl)-N-methyl-4-[4-(2-hydroxyethyl)isopropylaminomethylphenyl]cyclohexylamin

K = Cis-N-(4-Chlorbenzoyl)-N-methyl-4-[4-(3-hydroxypropyl)methylaminomethylphenyl]cyclohexylamin

L = trans-4-[4-(3-Hydroxypropyl)methylaminomethylphenyl]-N-methyl-N-(4-trifluormethylbenzoyl)-cyclohexylamin

M = trans-N-Cyclohexylcarbonyl-N-methyl-4-[4-(3-hydroxypropyl)methylaminomethylphenyl]-cyclohexylamin.

N = trans-N-(4-Chlor-3-methylbenzoyl)-N-methyl-4-[4-(3-hydroxypropyl)methylaminomethylphenyl]-cyclohexylamin

O = trans-N-(3,4-Dichlorphenylacetyl)-N-methyl-4-[4-(3-hydroxypropyl)methylaminomethylphenyl]-cyclohexylamin

P = trans-4-[4-(3-Hydroxypropyl)methylaminomethylphenyl]-N-methyl-N-(1,2,3,4-tetrahydronaphthalin-2-carbonyl)cyclohexylamin

Q = trans-N-(5-Chlorthienyl-2-carbonyl)-N-methyl-4-[4-(3-hydroxypropyl)methylaminomethylphenyl]-cyclohexylamin

Die Prozentwerte, um die die obigen Verbindungen den $^{14}C$-Acetat-Einbau hemmen, sind in der folgenden Tabelle angegeben:

| Mol/l | $10^{-7}$ | $10^{-8}$ |
|---|---|---|
| A | -88 | -80 |
| B | -82 | -77 |
| C | -77 | -36 |
| D | -77 | -47 |
| E | -75 | -40 |
| F | -82 | -61 |
| G | -86 | -61 |
| H | -88 | -58 |
| I | -87 | -60 |
| K | -76 | -35 |
| L | -83 | -50 |
| M | -77 | -41 |
| N | -78 | -40 |
| O | -77 | -49 |
| P | -73 | -37 |
| Q | -80 | -62 |

Wie eingangs erwähnt, sind in der Literatur vereinzelt Inhibitoren des Enzyms 2,3-Epoxisqualen-Lanosterol-Cyclase beschrieben, die sich jedoch strukturell sehr stark von den erfindungsgemäßen Verbindungen der Formel I unterscheiden. Die zu den Verbindungen der allgemeinen Formel I strukturell nächstverwandten Verbindungen sind in der EP 0 468 457 beschrieben. Zum Vergleich wurde deshalb das Beispiel 1 dieser Publikation nach der oben beschriebenen Bestimmungsmethode in Testkonzentrationen von $10^{-5}$ Mol/l und $10^{-6}$ Mol/l geprüft. Die dabei gefundenen Hemmwerte von 41% bzw. 13% zeigen, daß diese Verbindungen den erfindungsgemäßen Verbindungen der allgemeinen Formel I deutlich unterlegen sind.

II. Messung der in-vivo-Wirkung an der Ratte nach oraler Gabe

Die Inhibierung des Enzyms 2,3-Epoxisqualen-Lanosterol-Cyclase bewirkt eine Erhöhung der 2,3-Epoxisqualenspiegel in Leber und Plasma. Die Menge an gebildetem 2,3-Epoxisqualen dient daher als direktes Maß für die Wirkstärke am Ganztier. Die Bestimmung wird nach folgender Methode durchgeführt: Männlichen Wistar-Ratten (160-190 g Körpergewicht) wird die in 1,5%iger wässriger Methylcellulose suspendierte Prüfsubstanz via Schlundsonde appliziert. 5 Stunden nach Applikation wird Blut retroorbital aus dem Venenplexus gewonnen. Plasma wird nach der Methode von Bligh und Dyer (Canad. J. Biochem. Physiol. 37, 912, [1959]) aufgearbeitet, über eine Vorsäule gereinigt und danach mittels HPLC analysiert. Die erhaltenen Peaks werden über Eichsubstanzen identifiziert und quantifiziert. Ein interner Standard dient der Überprüfung der Reproduzierbarkeit der Ergebnisse.

Die Untersuchungen wurden mit Konzentrationen von 0,1 bzw. 1,0 mg/kg durchgeführt. In der folgenden Tabelle sind beispielhaft die Ergebnisse der vorstehend erwähnten Substanzen A bis I zusammengefaßt: 2,3-Epoxisqualen-Konzentration ($\mu$g/ml) im Plasma (Ratte)

| mg/kg | 0,1 | 1,0 |
|---|---|---|
| A | 1,35 | 4,55 |
| B | 1,28 | 4,83 |
| C | 0,75 | 3,01 |
| D | 0,71 | 4,11 |
| E | 1,01 | 4,37 |
| F | 0,73 | 3,07 |
| G | 0,41 | 1,42 |
| H | 0,79 | 1,46 |
| I | 1,50 | 1,71 |

Bei den Kontrolltieren treten unter den Versuchsbedingungen keine meßbaren 2,3-Epoxisqualenspiegel auf.

Von keinem der in der Literatur beschriebenen Inhibitoren des Enzyms 2,3-Epoxisqualen-Lanosterol-Cyclase ist bisher eine Inhibierung der Cholesterolbiosynthese am Ganztier beschrieben.

III. Lipidsenkung am normolipämischen Goldhamster

Männliche Goldhamster werden für 12 Tage ad lib. mit cholesterolfreier Hamsterdiät gefüttert. Die zu testende Substanz wird dem Futter in Konzentrationen von 0,01 bis 0,10% zugemischt. Am Ende der Versuchsperiode werden das Gesamtcholesterol, die HDL-Fraktion sowie die VLDL + LDL-Fraktion nach Standardmethoden bestimmt, wobei zum Vergleich eine ohne Testsubstanz gefütterte Kontrollgruppe herangezogen wird.

Geprüft wurde die lipidsenkende Wirkung der vorstehend erwähnten Verbindung D. Das Ergebnis ist in der folgenden Tabelle zusammengefaßt:

| Konzentration | Gesamt-Cholesterol | VLDL + LDL | HDL |
|---|---|---|---|
| 0,01% | -21,8% | -28,8% | -17,3% |
| 0,03% | -28,6% | -42,9% | -23,6% |
| 0,10% | -35,1% | -42,5% | -30,6% |

Die Verbindungen A bis I zeigten sich in der kurativen Dosierung als untoxisch. Beispielsweise zeigten die Verbindungen D und H nach oraler Applikation von 100 mg/kg, einmal täglich über 4 Tage, an der Maus keine toxischen Wirkungen.

Zur pharmazeutischen Anwendung lassen sich die Verbindungen der allgemeinen Formel I in an sich bekannter Weise in die üblichen pharmazeutischen Zubereitungsformen für die orale, rektale und topische Verabreichung einarbeiten.

Formulierungen für die orale Verabreichung umfassen beispielsweise Tabletten, Dragées und Kapseln, für die rektale Verabreichnung kommen vorzugsweise Suppositorien in Betracht. Die Tagesdosis beträgt zwischen 1 und 1200 mg für einen Menschen mit 60 kg Körpergewicht, bevorzugt ist jedoch eine Tagesdosis von 5 bis 100 mg für einen Menschen mit 60 kg Körpergewicht. Die Tagesdosis wird vorzugsweise in 1 bis 3 Einzelgaben aufgeteilt.

Bei topischer Anwendung können die Verbindungen in Zubereitungen, die etwa 1 bis 1000 mg, insbesondere 10 bis 300 mg Wirkstoff pro Tag enthalten, verabreicht werden. Die Tagesdosis wird vorzugsweise in 1 bis 3 Einzelgaben aufgeteilt.

Topische Formulierungen umfassen Gele, Cremes, Lotionen, Salben, Puder, Aerosole und andere herkömmliche Formulierungen zur Anwendung von Heilmitteln auf der Haut. Die Wirkstoffmenge für die topische Anwendung beträgt 1 bis 50 mg pro Gramm Formulierung, vorzugsweise jedoch 5 bis 20 mg pro Gramm Formulierung. Neben der Anwendung auf der Haut können die topischen Formulierungen der vorliegenden Erfindung auch angewandt werden bei der Behandlung von Schleimhäuten, die der topischen Behandlung zugänglich sind. Beispielsweise können die topischen Formulierungen auf die Schleimhäute des Mundes, des unteren Colons und andere aufgebracht werden.

Zur Anwendung in der Geflügelzucht zur Erzeugung cholesterolarmer Eier werden die Wirkstoffe der allgemeinen Formel I den Tieren nach den üblichen Methoden als Zusatz zu geeigneten Futtermitteln verabreicht. Die Konzentration der Wirkstoffe im Fertigfutter beträgt normalerweise 0,01 bis 1%, vorzugsweise jedoch 0,05 bis 0,5%.

Die Wirkstoffe können als solche dem Futter zugesetzt werden. So enthalten die erfindungsgemäßen Futtermittel für Legehennen neben dem Wirkstoff und gegebenenfalls neben einer üblichen Vitamin-Mineral-Mischung beispielsweise Mais, Sojabohnenmehl, Fleischmehl, Futterfett und Sojaöl. Zu diesem Futter wird eine der eingangs erwähnten Verbindungen der Formel I als Wirkstoff in einer Konzentration von 0,01 bis 1%, vorzugsweise jedoch 0,05 bis 0,5% zugemischt.

Die nachfolgenden Beispiele dienen der näheren Erläuterung der Erfindung. Die angegebenen $R_f$-Werte wurden an Fertigplatten der Firma E. Merck, Darmstadt bestimmt und zwar an:

a) Aluminiumoxid F-254 (Typ E)

b) Kieselgel 60 F-254.

Beispiele zur Herstellung der Ausgangsmaterialien

Beispiel A

4-(4-tert.Butoxycarbonyl-methylaminomethylphenyl)cyclohexanon

Zu 100 g 4-Brombenzaldehyd und 300 g Molekularsieb (3 Angström) werden unter Eiskühlung 800 ml einer 8%igen Lösung von Methylamin in Toluol gegeben. Das Gemisch wird über Nacht bei Raumtemperatur gerührt, vom Molekularsieb abgesaugt und das Lösungsmittel verdampft. Der Rückstand wird in 1,4 l eiskaltem Methanol gelöst und unter Eiskühlung werden 48 g Natriumborhydrid portionsweise zugegeben. Danach wird 30 Minuten bei 0 ° C, eine Stunde bei 10 ° C und zwei Stunden bei Raumtemperatur gerührt, zur Trockne eingedampft, der Rückstand in Eiswasser aufgenommen, mit halbkonzentrierter Salzsäure angesäuert und zweimal mit Ether extrahiert. Der Ether wird verworfen, die wässrige Phase unter Kühlung mit konzentrierter Natronlauge alkalisch gestellt und dreimal mit Ether extrahiert. Die organische Phase wird mit Wasser und gesättigter Kochsalzlösung gewaschen, getrocknet und eingedampft. Man erhält 96,8 g N-Methyl-4-brombenzylamin als farbloses Öl.

Dieses Produkt wird in 466 ml wasserfreiem Tetrahydrofuran gelöst und es werden bei -15 bis -20 ° C 300 ml einer 1,6 M Lösung von n-Butyllithium in Hexan zugetropft. Unmittelbar danach werden bei derselben Temperatur 52,5 g Trimethylchlorsilan zugetropft und 10 Minuten gerührt. Dann werden bei -75 bis -65 ° C weitere 320 ml der oben genannten n-Butyllithiumlösung zugetropft, es wird weitere 20 Minuten bei -75 ° C gerührt und anschließend bei -75 bis -65 ° C eine Lösung von 76 g 1,4-Cyclohexandionmonoethylenketal in 225 ml wasserfreiem Tetrahydroduran zugetropft. Nach 30 Minuten Rühren bei dieser Temperatur läßt man auf Raumtemperatur erwärmen, gießt die Reaktionsmischung in Wasser und extrahiert dreimal mit Essigsäureethylester. Die organische Phase wird mit Wasser und gesättigter Kochsalzlösung gewaschen, getrocknet und eingedampft. Der Rückstand wird aus Ether kristallisiert. Man erhält 87,1 g 4-Hydroxy-4-(4-methylaminomethylphenyl)cyclohexanon-ethylenketal vom Schmelzpunkt 98-100 ° C.

Dieses Produkt wird zusammen mit 65,7 g p-Toluolsulfonsäure in 1200 ml Toluol und 200 ml Ethylenglykol bis zum Ende der Wasserabscheidung am Wasserabscheider zum Rückfluß erhitzt (ca. 2,5 Stunden), abgekühlt und die Lösung mit Essigsäureethylester verdünnt. Es wird mit Natronlauge, Wasser und gesättigter Kochsalzlösung gewaschen, getrocknet und eingedampft. Der ölige Rückstand wird in 700 ml wasserfreiem Tetrahydrofuran gelöst, portionsweise mit 75 g Pyrokohlensäuredi-tert.butylester versetzt und zwei Stunden bei Raumtemperatur gerührt. Das Lösungsmittel wird verdampft, der Rückstand in Ether gelöst, mit Wasser gewaschen, getrocknet und der Ether verdampft. Man erhält 122 g 4-(4-tert.Butoxycarbonyl-methylaminomethylphenyl)cyclohex-3-enon-ethylenketal als farbloses Öl.

Dieses Produkt wird in einem Gemisch aus 400 ml Methanol und 400 ml Essigsäureethylester gelöst und in Gegenwart von 15 g Palladium/Bariumsulfat bei Raumtemperatur und 5 Bar Wasserstoffdruck hydriert. Es wird vom Katalysator abfiltriert und das Lösungsmittel verdampft. Der Rückstand wird aus Diisopropylether bei tiefer Temperatur umkristallisiert. Man erhält 88 g 4-(4-tert.Butoxycarbonyl-methylaminomethylphenyl)cyclohexanon-ethylenketal vom Schmelzpunkt 117-118 ° C.

47,5 g dieses Produkts werden zusammen mit 900 ml Aceton, 90 ml Wasser und 4,5 g Pyridinium-p-toluolsulfonat 16 Stunden am Rückfluß erhitzt. Nach Abkühlen und Zugabe von Wasser wird das Aceton verdampft und die wäßrige Mischung mit Essigsäureethylester versetzt. Die organische Phase wird zweimal mit Wasser extrahiert, mit gesättigter Kochsalzlösung gewaschen, getrocknet und eingedampft. Nach Umkristallisation aus Petrolether erhält man 34 g der Titelverbindung vom Schmelzpunkt 62-64 ° C.

Auf dieselbe Weise wurden erhalten:

a) 4-(3-tert.Butoxycarbonyl-methylaminomethylphenyl)cyclohexanon
aus 3-Brombenzaldehyd, Methylamin und 1,4-Cyclohexandionmonoethylenketal.
Farbloses Öl.
$R_f$-Wert: 0,38 (Kieselgel, Petrolether/Essigsäureethylester 3:1, v:v).

b) 4-[4-(1-tert.Butoxycarbonyl-methylaminoethyl)phenyl]cyclohexanon
aus 4-Bromacetophenon, Methylamin und 1,4-Cyclohexandionmonoethylenketal.
Öl.
$R_f$-Wert: 0,35 (Kieselgel, Petrolether/Essigsäureethylester 3:1, v:v).

Beispiel B

4-(4-Hydroxymethylphenyl)cyclohexanon

Zum Schutz der Hydroxygruppe werden zunächst 31 g (0,166 Mol) 4-Brombenzylalkohol nach literaturbekannten Methoden durch Umsetzung mit Dihydropyran in den entsprechenden Tetrahydropyranylether übergeführt (200 ml Tetrahydrofuran, 50 ml 1,2-Dihydropyran, katalytische Menge p-Toluolsulfonsäure, 48 Stunden Rühren bei Raumtemperatur). Das erhaltene Produkt wird, wie in Beispiel A beschrieben, mit n-Butyllithium und 1,4-Cyclohexandionmonoethylenketal umgesetzt. Man erhält 58 g (81% der Theorie) 4-Hydroxy-4-[4-(2-Pyranyloxymethylphenyl)cyclohexanon-ethylenketal vom Schmelzpunkt 99-101 °C.

3,5 g dieses Produkts in 30 ml Pyridin werden unter Eiskühlung mit 2,2 ml Thionylchlorid versetzt. Das erhaltene Öl (2,7 g), das ein Gemisch zweier Substanzen darstellt, wird in in einem Gemisch aus 40 ml Essigsäureethylester und 20 ml Methanol in Gegenwart von 1,5 g Palladium/Calciumcarbonat bei Raumtemperatur und einem Wasserstoffdruck von 5 bar hydriert. Das erhaltene Öl (2,4 g) wird in 40 ml Aceton und 10 ml Wasser mit 0,2 g Pyridium-p-Toluolsulfonat über Nacht zum Rückfluß erhitzt. Das Rohprodukt wird durch Säulenchromatographie( Kieselgel, Essigsäureethylester/Petrolether = 1:1, v:v) gereinigt. Man erhält 1,0 g der Titelverbindung vom Schmelzpunkt 58-60 °C.

Beispiel C

cis- und trans-4-(4-tert.Butoxycarbonyl-methylaminomethylphenyl)-N-methylcyclohexylamin

6,3 g (0,02 Mol) 4-(4-tert.Butoxycarbonyl-methylaminomethylphenyl)cyclohexanon werden auf die in Beispiel A für 4-Brombenzaldehyd beschriebene Weise mit Methylamin und Natriumborhydrid umgesetzt. Das erhaltene Produkt wird durch Säulenchromatographie gereinigt (Aluminiumoxid, Essigsäureethylester/Methanol = 20:1 bis 1:1, v:v). Man erhält zunächst eine geringe Menge des cis-Isomeren als farbloses Öl. $R_f$-Wert: 0,56 (Aluminiumoxid, Essigsäureethylester/Methanol = 24:1, v:v).

Anschließend erhält man als eine weitere Fraktion 4,0 g (60,2% der Theorie) des trans-Isomeren der Titelverbindung als farbloses Öl.

$R_f$-Wert: 0,34 (Aluminiumoxid, Essigsäureethylester/Methanol = 24:1, v:v).

Auf dieselbe Weise wurden erhalten:

a) trans-4-(4-tert.Butoxycarbonyl-methylaminomethylphenyl)-N-cyclopropylcyclohexlamin
aus 4-(4-tert.Butoxycarbonyl-methylaminomethylphenyl)cyclohexanon und Cyclopropylamin.
Farbloses Öl.
$R_f$-Wert: 0,51 (Aluminiumoxid, Petrolether/Essigsäureethylester = 4:1, v:v).

b) trans-N-Allyl-4-(4-tert.butoxycarbonyl-methylaminomethylphenyl)cyclohexylamin
aus 4-(4-tert.Butoxycarbonyl-methylaminomethylphenyl)cyclohexanon und Allylamin.
Farbloses Öl.
$R_f$-Wert: 0,52 (Aluminiumoxid, Petrolether/Essigsäureethylester = 3:1, v:v).

c) trans-4-(4-tert.Butoxycarbonyl-methylaminomethylphenyl)-N-propargylcyclohexylamin
aus 4-(4-tert.Butoxycarbonyl-methylaminomethylphenyl)cyclohexanon und Progargylamin.
Farbloses Öl.
$R_f$-Wert: 0,39 (Aluminiumoxid, Petrolether/Essigsäureethylester = 3:1, v:v).

d) trans-4-[4-(1-tert.Butoxycarbonyl-methylaminoethyl)phenyl]-N-methylcyclohexylamin
aus 4-[4-(1-tert.Butoxycarbonyl-methylaminoethyl)phenyl]cyclohexanon und Methylamin.
Öl.
$R_f$-Wert: 0,5 (Aluminiumoxid, Essigsäureethylester/Methanol = 10:1, v:v).

e) trans-4-(3-tert.Butoxycarbonyl-methylaminomethylphenyl)-N-methylcyclohexylamin
aus 4-(3-tert.Butoxycarbonyl-methylaminomethylphenyl)cyclohexanon und Methylamin.
Farbloses Öl.

f) trans-N-Methyl-4-phenylcyclohexylamin
aus 4-Phenylcyclohexanon und Methylamin.
Schmelzpunkt: 40 °C.

g) trans-N-Cyclohexyl-4-phenylcyclohexylamin
aus 4-Phenylcyclohexanon und Cyclohexylamin.
Schmelzpunkt: 68-70 °C.

h) trans-N-Isopropyl-4-phenylcyclohexylamin
aus 4-Phenylcyclohexanon und Isopropylamin.

Schmelzpunkt: 61-63 °C.
i) trans-N-Hexyl-4-phenylcyclohexylamin
aus 4-Phenylcyclohexanon und Hexylamin.
Farbloses Öl.
$R_f$-Wert: 0,25 (Aluminiumoxid, Petrolether/Essigsäureethylester = 9:1, v:v).
j) trans-N-Neopentyl-4-phenylcyclohexylamin
aus 4-Phenylcyclohexanon und Neopentylamin.
Farbloses Öl.
k) trans-4-(4-Hydroxymethylphenyl)-N-methylcyclohexylamin
aus 4-(4-Hydroxymethylphenyl)cyclohexanon und Methylamin.
Schmelzpunkt: 131-134 °C.
l) trans-4-Phenylcyclohexylamin
aus 4-Phenylcyclohexanon und Benzylamin, gefolgt von katalytischer Entfernung des N-Benzylrestes (Palladium/Kohle).
Schmelzpunkt: Sintern ab 46-48 °C.
m) trans-4-(4-tert.Butoxycarbonyl-methylaminomethylphenyl)-N-ethylcyclohexylamin
aus 4-(4-tert.Butoxycarbonyl-methylaminomethylphenyl)cyclohexanon und Ethylamin.
Farbloses Öl.
$R_f$-Wert: 0,45 (Aluminiumoxid, Petrolether/Essigsäureethylester = 1:1, v:v).
n) trans-N-Benzyl-4-(4-tert.butoxycarbonyl-methylaminomethylphenyl)cyclohexylamin
aus 4-(4-tert.Butoxycarbonyl-methylaminomethylphenyl)cyclohexanon und Benzylamin.
Farbloses Öl.
$R_f$-Wert: 0,45 (Aluminiumoxid, Petrolether/Essigsäureethylester = 2:1, v:v).
o) trans-4-(4-tert.Butoxycarbonyl-methylaminomethylphenyl)-N-(4-fluorbenzyl)cyclohexylamin
aus 4-(4-tert.Butoxycarbonyl-methylaminomethylphenyl)cyclohexanon und 4-Fluorbenzylamin.
Farbloses Öl.
$R_f$-Wert: 0,43 (Aluminiumoxid, Petrolether/Essigsäureethylester = 5:1, v:v).

## Beispiel D

### trans-4-(4-tert.Butoxycarbonyl-methylaminomethylphenyl)-N-(4-chlorbenzoyl)-N-methylcyclohexylamin

8 g (0,024 Mol) trans-4-(4-tert.Butoxycarbonyl-methylaminomethylphenyl)-N-methylcyclohexylamin werden in 300 ml Ether mit 4,6 g (0,026 Mol) 4-Chlorbenzoylchlorid in Gegenwart von 2,7 g (0,026 Mol) Triethylamin umgesetzt. Man erhält 10,3 g der Titelverbindung vom Schmelzpunkt 152-154 °C.

Auf dieselbe Weise wurden dargestellt:
a) trans-4-(4-tert.Butoxycarbonyl-methylaminomethylphenyl)-N-(4-chlorbenzoyl)-N-cyclopropylcyclohexylamin
aus trans-4-(4-tert.Butoxycarbonyl-methylaminomethylphenyl)-N-cyclopropylcyclohexlamin und 4-Chlorbenzoylchlorid.
Schmelzpunkt: 151-152 °C.
b) trans-N-Allyl-N-(4-chlorbenzoyl)-4-(4-tert.butoxycarbonyl-methylaminomethylphenyl)cyclohexylamin
aus trans-N-Allyl-4-(4-tert.butoxy-carbonylmethylaminomethylphenyl)cyclohexylamin und 4-Chlorbenzoylchlorid.
Farbloses Öl.
$R_f$-Wert: 0,43 (Aluminiumoxid, Petrolether/Essigsäureethylester = 2:1, v:v).
c) trans-4-(4-tert.Butoxycarbonyl-methylaminomethylphenyl)-N-(4-chlorbenzoyl)-n-propargylcyclohexylamin
aus trans-4-(4-tert.Butoxycarbonyl-methylaminomethylphenyl)-N-propargylcyclohexylamin und 4-Chlorbenzoylchlorid.
Farbloses Öl.
$R_f$-Wert: 0,46 (Kieselgel, Petrolether/Essigsäureethylester = 2:1, v:v).
d) trans-4-[4-(1-tert.Butoxycarbonyl-methylaminoethyl)phenyl]-N-(4-chlorbenzoyl)-N-methylcyclohexylamin
aus trans-4-[4-(1-tert.Butoxycarbonyl-methylaminoethyl)phenyl]-N-methylcyclohexylamin und 4-Chlorbenzoylchlorid.
Schmelzpunkt: 172-174 °C.

e) trans-4-(3-tert.Butoxycarbonyl-methylaminomethylphenyl)-N-(4-chlorbenzoyl)-N-methylcyclohexylamin
aus trans-4-(3-tert.Butoxycarbonyl-methylaminomethylphenyl)-N-methylcyclohexylamin und 4-Chlorbenzoylchlorid.
Schmelzpunkt: 134-135 ° C.
f) trans-N-(4-Chlorbenzoyl)-N-methyl-4-phenylcyclohexylamin
aus trans-N-Methyl-4-phenylcyclohexylamin und 4-Chlorbenzoylchlorid.
Schmelzpunkt: 138-140 ° C.
g) trans-N-(4-Chlorbenzoyl)-N-cyclohexyl-4-phenylcyclohexylamin
aus trans-N-Cyclohexyl-4-phenylcyclohexylamin und 4-Chlorbenzoylchlorid.
Schmelzpunkt: 164-166 ° C.
h) trans-N-(4-Chlorbenzoyl)-N-isopropyl-4-phenylcyclohexylamin
aus trans-N-Isopropyl-4-phenylcyclohexylamin und 4-Chlorbenzoylchlorid.
Schmelzpunkt: 130-132 ° C.
i) trans-N-(4-Chlorbenzoyl)-N-hexyl-4-phenylcyclohexylamin
aus trans-N-Hexyl-4-phenylcyclohexylamin und 4-Chlorbenzoylchlorid.
Schmelzpunkt: 105 ° C.
j) trans-N-(4-Chlorbenzoyl)-N-neopentyl-4-phenylcyclohexylamin
aus trans-N-Neopentyl-4-phenylcyclohexylamin und 4-Chlorbenzoylchlorid.
Farblose Kristalle.
k) trans-N-(4-Chlorbenzoyl)-N-methyl-4-(4-hydroxymethylphenyl)cyclohexylamin
aus trans-4-(4-Hydroxymethylphenyl)-N-methylcyclohexylamin und 4-Chlorbenzoylchlorid.
Schmelzpunkt: 211-213 ° C.
l) trans-N-(4-Chlorbenzoyl)-4-phenylcyclohexylamin
aus trans-4-Phenylcyclohexylamin und 4-Chlorbenzoylchlorid.
Schmelzpunkt: 232-234 ° C.
m) trans-4-(4-tert.Butoxycarbonyl-methylaminomethylphenyl)-N-(4-chlorbenzolsulfonyl)-N-methylcyclohexylamin
aus trans-4-(4-tert.Butoxycarbonyl-methylaminomethylphenyl)-N-methylcyclohexylamin und 4-Chlorbenzolsulfonsäurechlorid.
Schmelzpunkt: 105-106 ° C.
n) trans-N-Acetyl-N-benzyl-4-(4-tert.Butoxycarbonyl-methylaminomethylphenyl)cyclohexylamin
aus trans-N-Benzyl-4-(4-tert.butoxycarbonyl-methylaminomethylphenyl)cyclohexylamin und Acetylchlorid.
Öl.
$R_f$-Wert: 0,66 (Aluminiumoxid, Essigsäureethylester/Petrolether = 1:3, v:v).
o) trans-N-Acetyl-N-(4-fluorbenzyl)-4-(4-tert.butoxycarbonylmethylaminomethylphenyl)cyclohexylamin
aus trans-4-(4-tert.Butoxycarbonyl-methylaminomethylphenyl)-N-(4-fluorbenzyl)cyclohexylamin und Acetylchlorid.
Farbloses Öl.
p) trans-4-(4-tert.Butoxycarbonyl-methylaminomethylphenyl)-N-(4-fluorbenzyl)-N-methansulfonylcyclohexylamin
aus trans-4-(4-tert.Butoxycarbonyl-methylaminomethylphenyl)-N-(4-fluorbenzyl)cyclohexylamin und Methansulfonsäurechlorid.
Farbloses Öl.
q) trans-4-(4-tert.Butoxycarbonyl-methylaminomethylphenyl)-N-methyl-N-(4-phenyl-3-butenoyl)-cyclohexylamin
aus trans-4-(4-tert.Butoxycarbonyl-methylaminomethylphenyl)-N-methylcyclohexylamin und 4-Phenyl-3-butenoylchlorid.
Farbloses Öl.
$R_f$-Wert: 0,5 (Kieselgel, Petrolether/Essigsäureethylester = 1:1, v:v).
r) trans-4-(4-tert.Butoxycarbonyl-methylaminomethylphenyl)-N-ethyl-N-(4-phenyl-3-butenoyl)-cyclohexylamin
aus trans-4-(4-tert.Butoxycarbonyl-methylaminomethylphenyl)-N-ethylcyclohexylamin und 4-Phenyl-3-butenoylchlorid.
Farbloses Öl.
$R_f$-Wert: 0,74 (Aluminiumoxid, Petolether/Essigsäureethylester = 2:1, v:v).
s) trans-N-Benzyl-N-(4-phenyl-3-butenoyl)-4-(4-tert.Butoxycarbonyl-methylaminomethylphenyl)-cyclohexylamin
aus trans-N-Benzyl-4-(4-tert.butoxycarbonyl-methylaminomethylphenyl)cyclohexylamin und 4-Phenyl-3-butenoylchlorid.
Farbloses Öl.
$R_f$-Wert: 0,26 (Aluminiumoxid, Petrolether/Essigsäureethylester/Methanol = 10:10:1, v:v:v).
t) cis-4-(4-tert.Butoxycarbonyl-methylaminomethylphenyl)-N-(4-chlorbenzoyl)-N-methylcyclohexylamin
aus cis-4-(4-tert.Butoxycarbonyl-methylaminomethylphenyl)-N-methylcyclohexylamin und 4-Chlorbenzoylchlorid.
Schmelzpunkt: 98-100 °C.
u) trans-4-(4-tert.Butoxycarbonyl-methylaminomethylphenyl)-N-hexanoyl-N-methylcyclohexylamin
aus trans-4-(4-tert.Butoxycarbonyl-methylaminomethylphenyl)-N-methylcyclohexylamin und Hexansäurechlorid.
Schmelzpunkt: 83-85 °C.
v) trans-4-[4-(1-tert.Butoxycarbonyl-methylaminoethyl)phenyl]-N-(4-chlorbenzolsulfonyl)-N-methylcyclohexylamin
aus trans-4-[4-(1-tert.Butoxycarbonyl-methylaminoethyl)phenyl]-N-methylcyclohexylamin und 4-Chlorbenzolsulfonsäurechlorid.
Schmelzpunkt: 55 °C.

Beispiel E

trans-N-tert.Butoxycarbonyl-N-methyl-4-(4-hydroxymethylphenyl)cyclohexylamin

15,3 g trans-4-(4-Hydroxymethylphenyl)-N-methylcyclohexylamin werden in 250 ml Tetrahydrofuran mit 16,7 g Pyrokohlensäure-di-tert.butylester umgesetzt. Man erhält 17,7 g der Titelverbindung vom Schmelzpunkt 111-113 °C.

Beispiel F

trans-N-(4-Chlorbenzoyl)-N-ethyl-4-phenylcyclohexylamin

1 g trans-N-(4-Chlorbenzoyl)-4-phenylcyclohexylamin in 20 ml Dimethylformamid werden mit 0,14 g 55%igem Natriumhydrid versetzt und nach 15 Minuten werden 0,62 g Ethyljodid Zugegeben. Nach 1 Stunde bei Raumtemperatur wird die Reaktionslösung eingeengt, der Rückstand in Wasser aufgenommen, mit Essigsäureethylester extrahiert, die organische Phase getrocknet und eingeengt. Nach Reinigung durch Säulenchromatographie (Kieselgel, Petrolether/Essigsäureethylester = 4:1 bis 3:1, v:v) erhält man 0,73 g der Titelverbindung vom Schmelzpunkt 127-129 °C.

Beispiel G

trans-N-(4-Chlorbenzoyl)-N-methyl-4-(4-chlormethylphenyl)cyclohexylamin

33,8 g trans-N-(4-Chlorbenzoyl)-N-methyl-4-phenylcyclohexylamin, 24,3 g Paraformaldehyd und 24,3 g Zinkchlorid werden bei Raumtemperatur in 1300 ml Methylenchlorid suspendiert und es wird während 2,5 Stunden Chlorwasserstoff eingeleitet, wobei die Temperatur auf 29 °C steigt. Die erhaltene Lösung wird über Nacht gerührt, in 1500 ml Eiswasser gegossen und anschließend bis zur Phasentrennung gerührt. Die organische Phase wird mit Wasser gewaschen, getrocknet, eingedampft und der Rückstand aus Essigsäureethylester umkristallisiert. Man erhält 30,1 g der Titelverbindung vom Schmelzpunkt 174-176 °C.

Auf dieselbe Weise wurden erhalten:
a) trans-N-(4-Chlorbenzoyl)-N-cyclohexyl-4-(4-chlormethylphenyl)cyclohexylamin
aus trans-N-(4-Chlorbenzoyl)-N-cyclohexyl-4-phenylcyclohexylamin und Paraformaldehyd.
Schmelzpunkt: 205-209 °C.
b) trans-N-(4-Chlorbenzoyl)-N-isopropyl-4-(4-chlormethylphenyl)cyclohexylamin
aus trans-N-(4-Chlorbenzoyl)-N-isopropyl-4-phenylcyclohexylamin und Paraformaldehyd.
Schmelzpunkt: 160-163 °C.
c) trans-N-(4-Chlorbenzoyl)-N-hexyl-4-(4-chlormethylphenyl)cyclohexylamin
aus trans-N-(4-Chlorbenzoyl)-N-hexyl-4-phenylcyclohexylamin und Paraformaldehyd.
Schmelzpunkt: 119 °C.
d) trans-N-(4-Chlorbenzoyl)-N-neopentyl-4-(4-chlormethylphenyl)cyclohexylamin
aus trans-N-(4-Chlorbenzoyl)-N-neopentyl-4-phenylcyclohexylamin und Paraformaldehyd.

$R_f$-Wert: 0,49 (Kieselgel, Petrolether/Essigsäureethylester = 4:1, v:v).
e) trans-N-(4-Chlorbenzoyl)-4-(4-chlormethylphenyl)cyclohexylamin
aus trans-N-(4-Chlorbenzoyl)-4-phenylcyclohexylamin und Paraformaldehyd.
Schmelzpunkt: 240 °C, sintert ab 230 °C.
f) trans-N-(4-Chlorbenzoyl)-N-ethyl-4-(4-chlormethylphenyl)cyclohexylamin
aus trans-N-(4-Chlorbenzoyl)-N-ethyl-4-phenylcyclohexylamin und Paraformaldehyd.
Farbloses Öl.
$R_f$-Wert: 0,38 (Kieselgel, Petrolether/Essigsäureethylester = 3:1, v:v).

Beispiel H

trans-4-(4-Brommethylphenyl)-N-(4-chlorbenzoyl)-N-methylcyclohexylamin

710 mg trans-N-(4-Chlorbenzoyl)-N-methyl-4-(4-hydroxymethylphenyl)cyclohexylamin werden in 20 ml Methylenchlorid mit 723 mg Tetrabrommethan und 572 mg Triphenylphosphin umgesetzt. Man erhält 250 mg der Titelverbindung vom Schmelzpunkt 181-183 °C.

Beispiel I

trans-N-tert.Butoxycarbonyl-N-methyl-4-(4-methansulfonyloxymethylphenyl)cyclohexylamin

15,4 g trans-N-tert.Butoxycarbonyl-N-methyl-4-(4-hydroxymethylphenyl)cyclohexylamin in 120 ml Methylenchlorid werden bei 0 °C mit 4,5 ml Methansulfonsäurechlorid versetzt und anschließend werden langsam 8,1 ml Triethylamin in 20 ml Methylenchlorid zugetropft. Nach Rühren über Nacht wird Wasser zugegeben, die organische Phase mit Wasser gewaschen, getrocknet und eingedampft. Nach Umkristallisieren aus Diisopropylether bei tiefer Temperatur erhält man 12,1 g der Titelverbindung vom Schmelzpunkt 138-139 °C.

Beispiel K

trans-N-tert.Butoxycarbonyl-N-methyl-4-[4-(3-hydroxypropyl)methylaminomethylphenyl]cyclohexylamin

6,0 g trans-N-tert.Butoxycarbonyl-N-methyl-4-(4-methansulfonyloxymethylphenyl)cyclohexylamin, 4,2 g Kaliumcarbonat und 1,6 g N-Methyl-3-hydroxypropylamin werden zusammen in 50 ml Dimethylformamid über Nacht auf 50 °C erwärmt. Das Gemisch wird in Wasser gegossen und mit Essigsäureethylester extrahiert. Nach dem Trocknen der organischen Phase, Entfernen des Lösungsmittels und Umkristallisieren aus Petrolether erhält man 7,1 g der Titelverbindung vom Schmelzpunkt 63-65 °C.

Beispiel L

trans-4-[4-(3-Hydroxypropyl)methylaminomethylphenyl]-N-methylcyclohexylamin

7,1 g trans-N-tert.Butoxycarbonyl-N-methyl-4-[4-(3-hydroxypropyl)methylaminomethylphenyl]-cyclohexylamin werden in 150 ml Methylenchlorid bei Raumtemperatur mit 20 ml Trifluoressigsäure versetzt. Nach 2 Stunden werden Eis und 6N Natronlauge zugegeben, die organische Phase abgetrennt, gewaschen, getrocknet und eingedampft. Nach Umkristallisieren des Rückstands aus Ether erhält man 3,1 g der Titelverbindung vom Schmelzpunkt 144-146 °C.

Beispiel M

trans-N-(4-Chlorbenzoyl)-N-methyl-4-(4-methylaminomethylphenyl)cyclohexylamin

10,3 g trans-4-(4-tert.Butoxycarbonyl-methylaminomethylphenyl)-N-(4-chlorbenzoyl)-N-methylcyclohexylamin werden, wie in Beispiel L beschrieben, mit Trifluoressigsäure umgesetzt. Man erhält 8,12 g der Titelverbindung vom Schmelzpunkt 149-151 °C.

Auf dieselbe Weise wurden dargestellt:
a) trans-N-(4-Chlorbenzoyl)-N-cyclopropyl-4-(4-methylaminomethylphenyl)cyclohexylamin
aus trans-4-(4-tert.Butoxycarbonyl-methylaminomethylphenyl)-N-(4-chlorbenzoyl)-N-cyclopropylcyclohexylamin und Trifluoressigsäure.
Zähes Harz.
b) trans-N-Allyl-N-(4-chlorbenzoyl)-4-(4-methylaminomethylphenyl)cyclohexylamin
aus trans-N-Allyl-N-(4-chlorbenzoyl)-4-(4-tert.butoxycarbonyl-methylaminomethylphenyl)cyclohexylamin und Trifluoressigsäure.
Farbloses Öl.
c) trans-N-(4-Chlorbenzoyl)-N-propargyl-4-(4-methylaminomethylphenyl)cyclohexylamin
aus trans-4-(4-tert.Butoxycarbonyl-methylaminomethylphenyl)-N-(4-chlorbenzoyl)-n-propargylcyclohexylamin und Trifluoressigsäure.
Zähes Öl.
d) trans-N-(4-Chlorbenzoyl)-N-methyl-4-[4-(1-methylaminoethyl)phenyl]cyclohexylamin
aus trans-4-[4-(1-tert.Butoxycarbonyl-methylaminoethyl)phenyl]-N-(4-chlorbenzoyl)-N-methylcyclohexylamin und Trifluoressigsäure.
Schmelzpunkt: 153-155 °C.
e) trans-N-(4-Chlorbenzoyl)-N-methyl-4-(3-methylaminomethylphenyl)cyclohexylamin
aus trans-4-(3-tert.Butoxycarbonyl-methylaminomethylphenyl)-N-(4-chlorbenzoyl)-N-methylcyclohexylamin und Trifluoressigsäure.
Schmelzpunkt: 118-120 °C.
f) trans-N-(4-Chlorbenzolsulfonyl)-N-methyl-4-(4-methylaminomethylphenyl)cyclohexylamin
aus trans-(4-tert.Butoxycarbonyl-methylaminomethylphenyl)-N-(4-chlorbenzolsulfonyl)-N-methylcyclohexylamin und Trifluoressigsäure.
Schmelzpunkt: 105-107 °C.
g) trans-N-Acetyl-N-benzyl-4-(4-methylaminomethylphenyl)cyclohexylamin
aus trans-N-Acetyl-N-benzyl-4-(4-tert.Butoxycarbonyl-methylaminomethylphenyl)cyclohexylamin und Trifluoressigsäure. Zähes Harz.
$R_f$-Wert: 0,53 (Aluminiumoxid, Essigsäureethylester/Methanol = 24:1, v:v).
h) trans-N-Acetyl-N-(4-fluorbenzyl)-4-(4-methylaminomethylphenyl)cyclohexylamin
aus trans-N-Acetyl-N-(4-fluorbenzyl)-4-(4-tert.butoxycarbonyl-methylaminomethylphenyl)cyclohexylamin und Trifluoressigsäure.
$R_f$-Wert: 0,30 (Aluminiumoxid, Methylenchlorid/Methanol = 40:1, v:v).
i) trans-N-(4-Fluorbenzyl)-N-methansulfonyl-4-(4-methylaminomethylphenyl)cyclohexylamin
aus trans-4-(4-tert.Butoxycarbonyl-methylaminomethylphenyl)-N-(4-fluorbenzyl)-N-methansulfonylcyclohexylamin und Trifluoressigsäure.
$R_f$-Wert: 0,38 (Aluminiumoxid, Methylenchlorid/Methanol = 40:1, v:v).
j) trans-4-(4-Methylaminomethylphenyl)-N-methyl-N-(4-phenyl-3-butenoyl)cyclohexylamin
aus trans-4-(4-tert.Butoxycarbonyl-methylaminomethylphenyl)-N-methyl-N-(4-phenyl-3-butenoyl)-cyclohexylamin und Trifluoressigsäure.
Öl.
$R_f$-Wert: 0,2 (Aluminiumoxid, Petrolether/Essigsäureethylester/Methanol = 10:10:3, v:v:v).
k) trans-N-Ethyl-N-(4-phenyl-3-butenoyl)-4-(4-methylaminomethylphenyl)cyclohexylamin
aus trans-4-(4-tert.Butoxycarbonyl-methylaminomethylphenyl)-N-ethyl-N-(4-phenyl-3-butenoyl)-cyclohexylamin und Trifluoressigsäure.
Öl.
$R_f$-Wert: 0,24 (Aluminiumoxid, Petrolether/Essigsäureethylester/Methanol = 10:10:1, v:v:v).
l) trans-N-Benzyl-N-(4-phenyl-3-butenoyl)-4-(4-methylaminomethylphenyl)cyclohexylamin
aus trans-N-Benzyl-N-(4-phenyl-3-butenoyl)-4-(4-tert.Butoxycarbonyl-methylaminomethylphenyl)-cyclohexylamin und Trifluoressigsäure.
Öl.
$R_f$-Wert: 0,4 (Aluminiumoxid, Petrolether/Essigsäureethylester/Methanol = 10:10:1, v:v:v).
m) cis-N-(4-Chlorbenzoyl)-N-methyl-4-(4-methylaminomethylphenyl)cyclohexylamin
aus cis-4-(4-tert.Butoxycarbonyl-methylaminomethylphenyl)-N-(4-chlorbenzoyl)-N-methylcyclohexylamin und Trifluoressigsäure.
Schmelzpunkt: 110-112 °C.
n) trans-N-Hexanoyl-N-methyl-4-(4-methylaminomethylphenyl)cyclohexylamin
aus trans-4-(4-tert.Butoxycarbonyl-methylaminomethylphenyl)-N-hexanoyl-N-methylcyclohexylamin und Trifluoressigsäure.
Schmelzpunkt: 42-44 °C.

o) trans-N-(4-Chlorbenzolsulfonyl)-N-methyl-4-[4-(1-methylaminoethyl)phenyl]cyclohexylamin
aus trans-4-[4-(1-tert.Butoxycarbonyl-methylaminoethyl)phenyl]-N-(4-chlorbenzolsulfonyl)-N-methylcyclohexylamin und Trifluoressigsäure.
Schmelzpunkt: 82-85 °C.

Beispiele zur Herstellung der Endprodukte:

Beispiel 1

trans-N-(4-Chlorbenzoyl)-N-methyl-4-[4-(3-hydroxypropyl)methylaminomethylphenyl]cyclohexylamin

5,0 g (0,013 Mol) trans-N-(4-Chlorbenzoyl)-N-methyl-4-(4-methylaminomethylphenyl)cyclohexylamin, 2,1 g (0,0148 Mol) Kaliumcarbonat und 2,1 g (0,015 Mol) 3-Brompropanol-1 werden zusammen in 30 ml Dimethylformamid 40 Stunden bei Raumtemperatur gerührt. Anschließend wird die Reaktionsmischung in Wasser gegossen und mit Essigsäureethylester extrahiert. Nach Waschen mit Wasser und gesättigter Kochsalzlösung wird eingedampft und der Rückstand durch Säulenchromatographie (Aluminiumoxid, Essigsäureethylester/Methanol = 70:1 bis 40:1, v:v) gereinigt. Man erhält 3,8 g (68% der Theorie) der Titelverbindung vom Schmelzpunkt 98-100 °C.
$^1$H-NMR-Spektrum (200 MHz, $CDCl_3$); Signale bei ppm:
1,35 (m,1H), 1,6-2,1 (m,9H), 2,2 (2s,3H), 2,35-2,55 (m,1H), 2,6 (t,2H), 2,8-3,1 (m,3H), 3,45 (s,2H), 3,75 (t,2H), 3,6 + 4,6 (t + m,1H), 7,0-7,5 (m,8H).

Das Hydrochlorid der obigen Verbindung wurde erhalten durch Zugabe von etherischer Salzsäure zu einer Lösung der Base in Aceton und anschließendem Fällen des Hydrochlorids mit Ether. Schmelzpunkt: 162-164 °C.

Auf dieselbe Weise wurden erhalten:

a) trans-4-(4-Aminocarbonylmethyl-methylaminomethylphenyl)-N-(4-chlorbenzolsulfonyl)-N-methylcyclohexlamin
aus trans-N-(4-Chlorbenzolsulfonyl)-N-methyl-4-(4-methylaminomethylphenyl)cyclohexylamin und Chloracetamid.
Schmelzpunkt: 133-135 °C.

b) trans-4-(4-Aminocarbonylmethyl-methylaminomethylphenyl)-N-(4-chlorbenzoyl)-N-methylcyclohexylamin
aus trans-N-(4-Chlorbenzoyl)-N-methyl-4-(4-methylaminomethylphenyl)cyclohexylamin und Chloracetamid.
Schmelzpunkt: 138-140 °C.

c) trans-N-(4-Chlorbenzolsulfonyl)-N-methyl-4-[4-(2-methoxyethyl)methylaminomethylphenyl]-cyclohexylamin
aus trans-N-(4-Chlorbenzolsulfonyl)-N-methyl-4-(4-methylaminomethylphenyl)cyclohexylamin und (2-Brommethyl)methylether.
Schmelzpunkt: 55-57 °C.

d) trans-N-(4-Chlorbenzoyl)-N-methyl-4-[4-(3-cyanopropyl)methylaminomethylphenyl]cyclohexylamin
aus trans-N-(4-Chlorbenzoyl)-N-methyl-4-(4-methylaminomethylphenyl)cyclohexylamin und 4-Brombutyronitril.
Schmelzpunkt: 112-114 °C.

e) trans-4-[4-(1-Aminocarbonylethyl)methylaminomethylphenyl]-N-(4-chlorbenzoyl)-N-methyl-cyclohexylamin
aus trans-N-(4-Chlorbenzoyl)-N-methyl-4-(4-methylaminomethylphenyl)cyclohexylamin und 2-Chlorpropionamid.
Schmelzpunkt: 160-162 °C.

f) cis-N-(4-Chlorbenzoyl)-N-methyl-4-[4-(3-hydroxypropyl)methylaminomethylphenyl]cyclohexylamin
aus cis-N-(4-Chlorbenzoyl)-N-methyl-4-(4-methylaminomethylphenyl)cyclohexylamin und 3-Hydroxypropylamin.
Schmelzpunkt: 62-64 °C.

g) trans-4-[4-(3-Cyanopropyl)methylaminomethylphenyl]-N-hexanoyl-N-methylcyclohexylamin
aus trans-N-Hexanoyl-N-methyl-4-(4-methylaminomethylphenyl)cyclohexylamin und 4-Brombutyronitril.
Farbloses Öl.
$R_f$-Wert: 0,56 (Aluminiumoxid, Essigsäureethylester/Petrolether = 1:2, v:v).

h) trans-N-Hexanoyl-N-methyl-4-[4-(2-methoxyethyl)methylaminomethylphenyl]cyclohexylamin
aus trans-N-Hexanoyl-N-methyl-4-(4-methylaminomethylphenyl)cyclohexylamin und (2-Bromethyl)-methylether.
Öl.
$R_f$-Wert: 0,68 (Aluminiumoxid, Essigsäureethylester/Petrolether = 1:1, v:v).
i) trans-N-(4-Chlorbenzoyl)-N-methyl-4-[4-(2-methoxyethyl)methylaminomethylphenyl)cyclohexylamin
aus trans-N-(4-Chlorbenzoyl)-N-methyl-4-(4-methylaminomethylphenyl)cyclohexylamin und (2-Bromethyl)methylether.
Schmelzpunkt: 127-129 °C.
j) trans-N-(4-Chlorbenzoyl)-N-methyl-4-(4-Dimethylaminocarbonylmethyl-methylaminomethylphenyl)-cyclohexylamin
aus trans-N-(4-Chlorbenzoyl)-N-methyl-4-(4-methylaminomethylphenyl)cyclohexylamin und N,N-Dimethylchloracetamid.
Schmelzpunkt: 95-97 °C.
k) trans-N-(4-Chlorbenzolsulfonyl)-N-methyl-4-[4-(2-hydroxyethyl)methylaminomethylphenyl]-cyclohexylamin
aus trans-N-(4-Chlorbenzolsulfonyl)-N-methyl-4-(4-methylaminomethylphenyl)cyclohexylamin und Bromethanol.
Schmelzpunkt: 76-78 °C.
l) trans-N-(4-Chlorbenzolsulfonyl)-N-methyl-4-[4-(3-hydroxypropyl)methylaminomethylphenyl]-cyclohexylamin
aus trans-N-(4-Chlorbenzolsulfonyl)-N-methyl-4-(4-methylaminomethylphenyl)cyclohexylamin und 3-Brom-1-propanol.
Schmelzpunkt: 69-71 °C.
m) trans-N-(4-Chlorbenzolsulfonyl)-N-methyl-4-[4-(3-cyanopropyl)methylaminomethylphenyl]-cyclohexylamin
aus trans-N-(4-Chlorbenzolsulfonyl)-N-methyl-4-(4-methylaminomethylphenyl)cyclohexylamin und 4-Brombutyronitril.
$R_f$-Wert: 0,29 (Aluminiumoxid, Petrolether/Essigsäureethylester = 3:1, v:v).
n) trans-N-(4-Chlorbenzolsulfonyl)-N-methyl-4-(4-Methylaminocarbonylmethyl-methylaminomethylphenyl)-cyclohexylamin
aus trans-N-(4-Chlorbenzolsulfonyl)-N-methyl-4-(4-methylaminomethylphenyl)cyclohexylamin und N-Methylchloracetamid.
Schmelzpunkt: 115-117 °C.
o) trans-N-(4-Chlorbenzolsulfonyl)-N-methyl-4-[4-(5-hydroxypentyl)methylaminomethylphenyl]-cyclohexylamin
aus trans-N-(4-Chlorbenzolsulfonyl)-N-methyl-4-(4-methylaminomethylphenyl)cyclohexylamin und 5-Chlor-1-pentanol.
Öl.
$R_f$-Wert: 0,71 (Aluminiumoxid, Essigsäureethylester/Methanol = 50:1, v:v).
p) trans-4-(4-Aminocarbonylmethyl-methylaminomethylphenyl)-N-hexanoyl-N-methylcyclohexylamin
aus trans-N-Hexanoyl-N-methyl-4-(4-methylaminomethylphenyl)cyclohexylamin und Chloracetamid.
Schmelzpunkt: 84-86 °C.
q) trans-N-Acetyl-N-benzyl-4-[4-(3-hydroxypropyl)methylaminomethylphenyl]cyclohexylamin
aus trans-N-Acetyl-N-benzyl-4-(4-methylaminomethylphenyl)cyclohexylamin und 3-Brom-1-propanol.
Öl.
$R_f$-Wert: 0,66 (Aluminiumoxid, Essigsäureethylester/Methanol = 50:1, v:v).
r) trans-N-Acetyl-N-benzyl-4-(4-aminocarbonylmethyl-methylaminomethylphenyl)cyclohexylamin
aus trans-N-Acetyl-N-benzyl-4-(4-methylaminomethylphenyl)cyclohexylamin und Chloracetamid.
Schmelzpunkt: 99-101 °C.
s) trans-4-(4-Aminocarbonylmethyl-methylaminomethylphenyl)-N-(4-fluorbenzyl)-N-methansulfonylcyclohexylamin
aus trans-N-(4-Fluorbenzyl)-N-methansulfonyl-4-(4-methylaminomethylphenyl)cyclohexylamin und Iodacetamid.
Schmelzpunkt: 114-116 °C.
t) trans-N-(4-Fluorbenzyl)-N-methansulfonyl-4-[4-(3-hydroxypropyl)methylaminomethylphenyl]-cyclohexylamin
aus trans-N-(4-Fluorbenzyl)-N-methansulfonyl-4-(4-methylaminomethylphenyl)cyclohexylamin und 3-Brom-1-propanol.
Schmelzpunkt: 79-81 °C.

u) trans-N-(4-Chlorbenzoyl)-N-cyclopropyl-4-[4-(3-hydroxypropyl)methylaminomethylphenyl]-cyclohexylamin
aus trans-N-(4-Chlorbenzoyl)-N-cyclopropyl-4-(4-methylaminomethylphenyl)cyclohexylamin und 3-Brom-1-propanol.
Schmelzpunkt: 118-123 °C.

v) trans-N-(4-Chlorbenzoyl)-N-propargyl-4-[4-(3-hydroxypropyl)methylaminomethylphenyl]cyclohexylamin
aus trans-N-(4-Chlorbenzoyl)-N-propargyl-4-(4-methylaminomethylphenyl)cyclohexylamin und 3-Brom-1-propanol.
Öl.
$R_f$-Wert: 0,53 (Aluminiumoxid, Methylenchlorid/Methanol = 50:1, v:v).

w) trans-N-Allyl-N-(4-chlorbenzoyl)-4-[4-(3-hydroxypropyl)methylaminomethylphenyl]cyclohexylamin
aus trans-N-Allyl-N-(4-chlorbenzoyl)-4-(4-methylaminomethylphenyl)cyclohexylamin und 3-Brom-1-propanol.
Öl.
$R_f$-Wert: 0,56 (Aluminiumoxid, Methylenchlorid/Methanol = 50:1, v:v).

x) trans-N-(4-Chlorbenzoyl)-N-methyl-4-[3-(3-hydroxypropyl)methylaminomethylphenyl]cyclohexylamin
aus trans-N-(4-Chlorbenzoyl)-N-methyl-4-(3-methylaminomethylphenyl)cyclohexylamin und 3-Brom-1-propanol.
Wachs.
$R_f$-Wert: 0,68 (Aluminiumoxid, Essigsäureethylester).

y) trans-N-(4-Chlorbenzoyl)-N-methyl-4-[4-(1-((3-hydroxypropyl))methylaminoethyl)phenyl]cyclohexylamin
aus trans-N-(4-Chlorbenzoyl)-N-methyl-4-[4-(1-methylaminoethyl)phenyl]cyclohexylamin und 3-Brom-1-propanol.
Schmelzpunkt: 88-90 °C.

z) trans-N-(4-Chlorbenzoyl)-N-methyl-4-[4-(1-((2-hydroxyethyl))methylaminoethyl)phenyl]cyclohexylamin
aus trans-N-(4-Chlorbenzoyl)-N-methyl-4-[4-(1-methylaminoethyl)phenyl]cyclohexylamin und Bromethanol.
Schmelzpunkt: 118-120 °C.

aa) trans-4-[4-(1-Aminocarbonylmethyl-methylaminoethyl)phenyl]-N-(4-chlorbenzoyl)-N-methylcyclohexylamin
aus trans-N-(4-Chlorbenzoyl)-N-methyl-4-[4-(1-methylaminoethyl)phenyl]cyclohexylamin und Iodacetamid.
Schmelzpunkt: 173-175 °C.

ab) trans-N-(4-Chlorbenzolsulfonyl)-N-methyl-4-[4-(1-((3-hydroxypropyl))methylaminoethyl)phenyl]-cyclohexylamin
aus trans-N-(4-Chlorbenzolsulfonyl)-N-methyl-4-[4-(1-methylaminoethyl)phenyl]cyclohexylamin und 3-Brom-1-propanol.
Öl.
$R_f$-Wert: 0,4 (Aluminiumoxid, Petrolether/Essigsäureethylester = 1:1, v:v).

ac) trans N-(4-Chlorbenzolsulfonyl)-N-methyl-4-[4-(1-((2-hydroxyethyl))methylaminoethyl)phenyl]-cyclohexylamin
aus trans-N-(4-Chlorbenzolsulfonyl)-N-methyl-4-[4-(1-methylaminoethyl)phenyl]cyclohexylamin und Bromethanol.
Schmelzpunkt: 80-82 °C.

ad) trans-4-[4-(1-Aminocarbonylmethyl-methylaminoethyl)phenyl]-N-(4-chlorbenzolsulfonyl)-N-methylcyclohexylamin
aus trans-N-(4-Chlorbenzolsulfonyl)-N-methyl-4-[4-(1-methylaminoethyl)phenyl]cyclohexylamin und Iodacetamid.
Schmelzpunkt: 170-172 °C.

ae) trans-4-[4-(2-Hydroxyethyl)methylaminomethylphenyl]-N-methyl-N-(4-phenyl-3-butenoyl)-cyclohexylamin
aus trans-4-(4-Methylaminomethylphenyl)-N-methyl-N-(4-phenyl-3-butenoyl)cyclohexylamin und Bromethanol.
Öl.
$R_f$-Wert: 0,4 (Aluminiumoxid, Petrolether/Essigsäureethylester/Methanol = 10:10:1, v:v:v).

af) trans-4-[4-(3-Hydroxypropyl)methylaminomethylphenyl]-N-methyl-N-(4-phenyl-3-butenoyl)-cyclohexylamin
aus trans-4-(4-Methylaminomethylphenyl)-N-methyl-N-(4-phenyl-3-butenoyl)cyclohexylamin und 3-Brom-1-propanol.
Öl.
$R_f$-Wert: 0,5 (Aluminiumoxid, Petrolether/Essigsäureethylester/Methanol = 10:10:1, v:v:v).

ag) trans-4-(4-Aminocarbonylmethyl-methylaminomethylphenyl)-N-methyl-N-(4-phenyl-3-butenoyl)-cyclohexylamin
aus trans-4-(4-Methylaminomethylphenyl)-N-methyl-N-(4-phenyl-3-butenoyl)cyclohexylamin und Chloracetamid.
Schmelzpunkt: 118-120 °C.

ah) trans-N-Ethyl-N-(4-phenyl-3-butenoyl)-4-[4-(3-hydroxypropyl)methylaminomethylphenyl]-cyclohexylamin
aus trans-N-Ethyl-N-(4-phenyl-3-butenoyl)-4-(4-methylaminomethylphenyl)cyclohexylamin und 3-Brom-1-propanol.
Öl.
$R_f$-Wert: 0,55 (Aluminiumoxid, Petrolether/Essigsäureethylester/Methanol = 10:10:1, v:v:v).

ai) trans-N-Benzyl-N-(4-phenyl-3-butenoyl)-4-[4-(3-hydroxypropyl)methylaminomethylphenyl]-cyclohexylamin
aus trans-N-Benzyl-N-(4-phenyl-3-butenoyl)-4-(4-methylaminomethylphenyl)cyclohexylamin und 3-Brom-1-propanol.
Öl.
$R_f$-Wert: 0,45 (Aluminiumoxid, Petrolether/Essigsäureethylester/Methanol = 10:10:0,5, v:v:v).

## Beispiel 2

### trans-4-[4-(3-Acetoxypropyl)methylaminomethylphenyl]-N-(4-chlorbenzoyl)-N-methyl-cyclohexylamin

2,3 g (5,4 mMol) trans-N-(4-Chlorbenzoyl)-N-methyl-4-[4-(3-hydroxypropyl)methylaminomethylphenyl]-cyclohexylamin und 1,1 ml (6 mMol) Ethyldiisopropylamin in 50 ml Ether werden unter Eiskühlung mit 0,5 g (6 mMol) Acetylchlorid versetzt und 3 Stunden bei Raumtemperatur gerührt. Nach Verdünnen der Reaktionsmischung mit Ether wird mit kalter verdünnter Natronlauge extrahiert, die Etherphase mit Wasser und gesättigter Kochsalzlösung gewaschen, getrocknet und eingedampft. Man erhält nach Reinigung durch Säulenchromatographie (Aluminiumoxid, Essigsäureethylester/Petrolether = 1:1, v:v) 1,6 g (63% der Theorie) der Titelverbindung vom Schmelzpunkt 100-102 °C.
$^1$H-NMR-Spektrum (200 MHz, $CDCl_3$); Signale bei ppm:
1,35 (m,1H), 1,6-2,1 (s+m,12H), 2,2 (s,3H), 2,4-2,55 (t+m,3H), 2,8-3,1 (m,3H), 3,4 (s,2H), 3,55 und 4,6 (2m,1H), 4,1 (t,2H), 7,0-7,45 (m,8H).

Auf dieselbe Weise wurden erhalten:

a) trans-4-[4-(3-Acetosypropyl)methylaminomethylphenyl]-N-acetyl-N-benzylcyclohexylamin
aus trans-N-Acetyl-N-benzyl-4-[4-(3-hydroxypropyl)methylaminomethylphenyl]cyclohexylamin und Acetylchlorid.
Öl.
$R_f$-Wert: 0,67 (Aluminiumoxid, Essigsäureethylester/Petrolether = 1:1, v:v).

b) trans-4-[4-(3-Ethoxycarbonyloxypropyl)methylaminomethylphenyl]-N-(4-chlorbenzoyl)-N-methylcyclohexylamin
aus trans-N-(4-Chlorbenzoyl)-N-methyl-4-[4-(3-hydroxypropyl)methylaminomethylphenyl]cyclohexylamin und Chlorameisensäureethylester.
Schmelzpunkt: 87-89 °C.

c) trans-N-(4-Chlorbenzoyl)-N-methyl-4-[4-(3-pivaloyloxypropyl)methylaminomethylphenyl]cyclohexylamin
aus trans-N-(4-Chlorbenzoyl)-N-methyl-4-[4-(3-hydroxypropyl)methylaminomethylphenyl]cyclohexylamin und Pivaloylchlorid.
Schmelzpunkt: 100-102 °C.

d) trans-N-(4-Chlorbenzoyl)-N-methyl-4-[4-(3-isobutyrylosypropyl)methylaminomethylphenyl]-cyclohexylamin
aus trans-N-(4-Chlorbenzoyl)-N-methyl-4-[4-(3-hydroxypropyl)methylaminomethylphenyl]cyclohexylamin und Isobutyrylchlorid.
Schmelzpunkt: 79-81 °C.

Beispiel 3

trans-N-(4-Chlorbenzolsulfonyl)-N-methyl-4-[4-(2-hydroxypropyl)methylaminomethylphenyl]cyclohexylamin

407 mg (1 mMol) trans-N-(4-Chlorbenzolsulfonyl)-N-methyl-4-(4-methylaminomethylphenyl)-cyclohexylamin und 75 mg (1,3 mMol) 1,2-Propylenoxid werden in 3 ml Methanol bei Raumtemperatur bis zur vollständigen Umsetzung gerührt. Nach Verdampfen des Lösungsmittels erhält man 320 mg (69% der Theorie) der Titelverbindung vom Schmelzpunkt 90-92 °C.
$^1$H-NMR-Spektrum (200 MHz, $CDCl_3$); Signale bei ppm:
1,1 (d,3H), 1,5-1,7 (m,6H), 1,8-2,0 (m,2H), 2,2 (s,3H), 2,25-2,45 (m,3H), 2,8 (s,3H), 3,35-3,7 (q,2H), 3,8-4,0 (m,2H), 7,1-7,8 (2q,8H).

Auf dieselbe Weise wurden dargestellt:

a) trans-N-(4-Chlorbenzoyl)-N-methyl-4-[4-(S-2,3-dihydroxy-2-methylpropyl)methylaminomethylphenyl]-cyclohexylamin
aus trans-N-(4-Chlorbenzoyl)-N-methyl-4-(4-methylaminomethylphenyl)cyclohexylamin und (S)-2-Methylglycidol.
Schmelzpunkt: 103-105 °C.

b) trans-N-(4-Chlorbenzoyl)-N-methyl-4-[4-(R-2,3-dihydroxy-2-methylpropyl)methylaminomethylphenyl]-cyclohexylamin
aus trans-N-(4-Chlorbenzoyl)-N-methyl-4-(4-methylaminomethylphenyl)cyclohexylamin und (R)-2-Methylglycidol.
Schmelzpunkt: 104-106 °C.

c) trans-4-[4-(S-2,3-Dihydroxypropyl)methylaminomethylphenyl]-N-(4-chlorbenzoyl)-N-methylcyclohexylamin
aus trans-N-(4-Chlorbenzoyl)-N-methyl-4-(4-methylaminomethylphenyl)cyclohexylamin und (S)-2-Glycidol.
Schmelzpunkt: 109-111 °C.

d) trans-4-[4-(R-2,3-Dihydroxypropyl)methylaminomethylphenyl]-N-(4-chlorbenzoyl)-N-methylcyclohexylamin
aus trans-N-(4-Chlorbenzoyl)-N-methyl-4-(4-methylaminomethylphenyl)cyclohexylamin und (R)-2-Glycidol.
Schmelzpunkt: 109-111 °C.

Beispiel 4

trans-(4-Chlorbenzolsulfonyl)-N-methyl-4-[4-(2-oxopropyl)methylaminomethylphenyl]cyclohexylamin

285 mg (0,613 mMol) trans-N-(4-Chlorbenzolsulfonyl)-N-methyl-4-[4-(2-hydroxypropyl)-methylaminomethylphenyl]cyclohexylamin werden nach Pfitzner-Moffatt oxidiert (380 mg (1,94 mMol) Dicyclohexylcarbodiimid, 35 mg (0,3 mMol) Trifluoressigsäure, 0,9 ml (12,8 mMol) Dimethylsulfoxid und 2 ml Benzol). Man erhält 160 mg (56% der Theorie) der Titelverbindung als farblose Kristalle vom Schmelz-punkt 98-100 °C.
$^1$H-NMR-Spektrum (200 MHz, $CDCl_3$); Signale bei ppm:
1,35 (m,1H), 1,5-2,0 (m,9H), 2,15 (s,2H), 2,3 (s,2H), 2,4 (m,1H), 2,8 (s,3H), 3,15 (s,2H), 3,55 (s,2H), 3,9 (m,1H), 7,1-7,7 (2q, 8H).

Beispiel 5

trans-N-(4-Chlorbenzoyl)-N-methyl-4-[4-(2-hydroxyethyl)isopropylaminomethylphenyl]cyclohexylamin

380 mg (1 mMol) trans-N-(4-Chlorbenzoyl)-N-methyl-4-(4-chlormethylphenyl)cyclohexylamin werden mit 280 mg (2 mMol) Kaliumcarbonat und 110 mg (1,07 mMol) 2-Isopropylaminoethanol in 3 ml Dimethylformamid, wie in Beispiel 1 beschrieben, umgesetzt. Man erhält 259 mg (58,5% der Theorie) der Titelverbindung als farblose Kristalle vom Schmelzpunkt 98-100 °C.

Auf dieselbe Weise wurden erhalten:

a) trans-N-(4-Chlorbenzoyl)-N-methyl-4-[4-(2-hydroxyethyl)-n-pentylaminomethylphenyl]cyclohexylamin
aus trans-N-(4-Chlorbenzoyl)-N-methyl-4-(4-chlormethylphenyl)cyclohexylamin und 2-n-Pentylaminoethanol.

Schmelzpunkt: 40 ° C.

b) trans-N-(4-Chlorbenzoyl)-4-[4-(3-hydroxypropyl)methylaminomethylphenyl]cyclohexylamin aus trans-N-(4-Chlorbenzoyl)-4-(4-chlormethylphenyl)cyclohexylamin und 3-Hydroxypropylmethylamin.

Schmelzpunkt: 157-160 ° C.

c) trans-N-(4-Chlorbenzoyl)-N-ethyl-4-[4-(3-hydroxypropyl)methylaminomethylphenyl]cyclohexylamin aus trans-N-(4-Chlorbenzoyl)-N-ethyl-4-(4-chlormethylphenyl)cyclohexylamin und 3-Hydroxypropylmethylamin.

Öl.

$R_f$-Wert: 0,72 (Aluminiumoxid, Essigsäureethylester).

d) trans-N-(4-Chlorbenzoyl)-N-cyclohexyl-4-[4-(3-hydroxypropyl)methylaminomethylphenyl]-cyclohexylamin aus trans-N-(4-Chlorbenzoyl)-N-cyclohexyl-4-(4-chlormethylphenyl)cyclohexylamin und 3-Hydroxypropylmethylamin.

Schaum.

$R_f$-Wert: 0,41 (Aluminiumoxid, Petrolether/Essigsäureethylester = 1:1, v:v).

e) trans-N-(4-Chlorbenzoyl)-N-isopropyl-4-[4-(3-hydroxypropyl)methylaminomethylphenyl]cyclohexylamin aus trans-N-(4-Chlorbenzoyl)-N-isopropyl-4-(4-chlormethylphenyl)cyclohexylamin und 3-Hydroxypropylmethylamin.

Öl.

$R_f$-Wert: 0,35 (Aluminiumoxid, Petrolether/Essigsäureethylester = 1:1, v:v).

f) trans-N-(4-Chlorbenzoyl)-N-neopentyl-4-[4-(3-hydroxypropyl)methylaminomethylphenyl]cyclohexylamin aus trans-N-(4-Chlorbenzoyl)-N-neopentyl-4-(4-chlormethylphenyl)cyclohexylamin und 3-Hydroxypropylmethylamin.

$R_f$-Wert: 0,58 (Petrolether/Essigsäureethylester = 1:1, v:v).

g) trans-N-(4-Chlorbenzoyl)-N-hexyl-4-[4-(3-hydroxypropyl)methylaminomethylphenyl]cyclohexylamin aus trans-N-(4-Chlorbenzoyl)-N-hexyl-4-(4-chlormethylphenyl)cyclohexylamin und 3-Hydroxypropylmethylamin.

Öl.

$R_f$-Wert: 0,53 (Petrolether/Essigsäureethylester = 1:1, v:v).

## Beispiel 6

### trans-4-[4-(3-Hydroxypropyl-methylaminomethyl)phenyl]-N-methyl-N-(4-trifluormethylbenzoyl)-cyclohexylamin

Zu 200 mg (0,69 mMol) trans-4-[4-(3-Hydroxypropyl)methylaminomethylphenyl]-N-methylcyclohexylamin und 0,1 ml (0,73 mMol) Triethylamin in 3 ml Methylenchlorid wird unter Eiskühlung eine Lösung von 145 mg (0,69 mMol) 4-Trifluormethylbenzoylchlorid in wenig Methylenchlorid zugetropft und 2 Stunden bei Raumtemperatur gerührt. Nach Verdünnen der Reaktionsmischung mit Essigsäureethylester wird mit Wasser, In-Natronlauge und gesättigter Kochsalzlösung gewaschen, getrocknet und eingedampft. Der Rückstand wird durch Säulenchromatographie (Aluminiumoxid, Essigsäureethylester(Petrolether = 1:1, v:v) gereinigt. Man erhält 130 mg (40,8% der Theorie) der Titelverbindung vom Schmelzpunkt 87-89 ° C.

Auf dieselbe Weise wurden erhalten:

a) trans-N-Cyclopropylcarbonyl-N-methyl-4-[4-(3-hydroxypropyl)methylaminomethylpheny]-cyclohexylamin aus trans-4-[4-(3-Hydroxypropyl)methylaminomethylphenyl]-N-methylcyclohexylamin und Cyclopropancarbonsäurechlorid.

Öl.

$R_f$-Wert: 0,45 (Aluminiumoxid, Methylenchlorid/Methanol = 40:1, v:v).

b) trans-N-Cyclohexylcarbonyl-N-methyl-4-[4-(3-hydroxypropyl)methylaminomethylpheny]cyclohexylamin aus trans-4-[4-(3-Hydroxypropyl)methylaminomethylphenyl]-N-methylcyclohexylamin und Cyclohexancarbonsäurechlorid.

Schmelzpunkt: 66-68 ° C.

c) trans-4-[4-(3-Hydroxypropyl)methylaminomethylphenyl]-N-methyl-N-stearoylcyclohexylamin aus trans-4-[4-(3-Hydroxypropyl)methylaminomethylphenyl]-N-methylcyclohexylamin und Stearinsäurechlorid.

Schmelzpunkt: 55-57 ° C.

d) trans-N-(4-Fluorbenzoyl)-N-methyl-4-[4-(3-hydroxypropyl)methylaminomethylphenyl]cyclohexylamin aus trans-4-[4-(3-Hydroxypropyl)methylaminomethylphenyl]-N-methylcyclohexylamin und 4-Fluorbenzoylchlorid.
Schmelzpunkt: 105-107 °C.
e) trans-N-(4-Chlor-3-methylbenzoyl)-N-methyl-4-[4-(3-hydroxypropyl)methylaminomethylphenyl]-cyclohexylamin
aus trans-4-[4-(3-Hydroxypropylmethylaminomethyl)phenyl]-N-methylcyclohexylamin und 4-Chlor-3-methylbenzoylchlorid.
Schmelzpunkt: 98-100 °C.
f) trans-4-[4-(3-Hydroxypropyl)methylaminomethylphenyl]-N-methyl-N-(2-naphthylacetyl)cyclohexylamin
aus trans-4-[4-(3-Hydroxypropylmethylaminomethyl)phenyl]-N-methylcyclohexylamin und 2-Naphthalinessigsäurechlorid.
Schmelzpunkt: 127-129 °C.
g) trans-4-[4-(3-Hydroxypropyl)methylaminomethylphenyl]-N-methyl-N-(1,2,3,4-tetrahydronaphthalin-2-carbonyl)-cyclohexylamin
aus trans-4-[4-(3-Hydroxypropylmethylaminomethyl)phenyl]-N-methylcyclohexylamin und 1,2,3,4-Tetrahydronaphthalin-2-carbonsäurechlorid.
Schmelzpunkt: 93-95 °C.
h) trans-4-[4-(3-Hydroxypropyl)methylaminomethylphenyl]-N-methyl-N-(5-methylthienyl-2-carbonyl)-cyclohexylamin
aus trans-4-[4-(3-Hydroxypropylmethylaminomethyl)phenyl]-N-methylcyclohexylamin und 5-Methylthiophen-2-carbonsäurechlorid.
Schmelzpunkt: 85-87 °C.
i) trans-N-(5-Chlorthienyl-2-carbonyl)-N-methyl-4-[4-(3-hydroxypropyl)methylaminomethylphenyl]-cyclohexylamin
aus trans-4-[4-(3-Hydroxypropylmethylaminomethyl)phenyl]-N-methylcyclohexylamin und 5-Chlorthiophen-2-carbonchlorid.
Schmelzpunkt: 92-94 °C.

## Beispiel 7

trans-N-(3,4-Dichlorphenylacetyl)-N-methyl-4-[4-(3-hydroxypropyl)methylaminomethylphenyl]cyclohexylamin

141 mg (0,68 mMol) 3,4-Dichlorphenylessigsäure in 5 ml Xylol werden mit 112 mg (0,54 mMol) N,N'-Dicyclohexylcarbodiimid versetzt und 1 Stunde auf 50 °C erwärmt. Nach Zugabe von 200 mg (0,68 mMol) trans-4-[4-(3-Hydroxypropyl)methylaminomethylphenyl]-N-methylcyclohexylamin wird über Nacht bei 140 °C gerührt. Nach Abkühlen auf Raumtemperatur und Zugabe von Essigsäureethylester wird mit Wasser und gesättigter Kochsalzlösung gewaschen, getrocknet und eingedampft. Nach Reinigung durch Säulenchromatographie (Aluminiumoxid, Essigsäureethylester/Methanol = 100:1 bis 10:1, v:v) erhält man 110 mg (34% der Theorie) der Titelverbindung vom Schmelzpunkt 82-84 °C.

Auf dieselbe Weise wurde erhalten:
a) trans-4-[4-(3-Hydroxypropyl)methylaminomethylphenyl]-N-methyl-N-(4-pentinoyl)cyclohexylamin
aus trans-4-[4-(3-Hydroxypropyl)methylaminomethylphenyl)-N-methylcyclohexylamin, N.N'-Carbonyldiimidazol und 4-Pentinsäure.
Öl.
$R_f$-Wert: 0,66 (Aluminiumoxid/Essigsäureethylester/Methanol = 100:1, v:v).

Im folgenden wird die Herstellung pharmazeutischer Anwendungsformen anhand einiger Beispiele beschrieben:

Beispiel I

Tabletten mit 5 mg trans-N-(4-Chlorbenzoyl)-N-methyl-4-[4-(3-hydroxypropyl)methylaminomethylphenyl]-cyclohexylamin

Zusammensetzung:

1 Tablette enthält:

| | |
|---|---|
| Wirkstoff | 5,0 mg |
| Milchzucker | 148,0 mg |
| Kartoffelstärke | 65,0 mg |
| Magnesiumstearat | 2,0 mg |
| | 220,0 mg |

Herstellungsverfahren:

Aus Kartoffelstärke wird durch Erwärmen ein 10%iger Schleim hergestellt. Die Wirksubstanz, Milchzucker und die restliche Kartoffelstärke werden gemischt und mit obigem Schleim durch ein Sieb der Maschenweite 1,5 mm granuliert. Das Granulat wird bei 45 °C getrocknet, nochmals durch obiges Sieb gerieben, mit Magnesiumstearat vermischt und zu Tabletten verpreßt.

| | |
|---|---|
| Tablettengewicht: | 220 mg |
| Stempel: | 9 mm |

Beispiel II

Dragées mit 5 mg trans-N-(4-Chlorbenzoyl)-N-methyl-4-[4-(3-hydroxypropyl)methylaminomethylphenyl]-cyclohexylamin

Die nach Beispiel I hergestellten Tabletten werden nach bekanntem Verfahren mit einer Hülle überzogen, die im wesentlichen aus Zucker und Talkum besteht. Die fertigen Dragées werden mit Hilfe von Bienenwachs poliert.
Dragéegewicht: 300 mg

Beispiel III

Suppositorien mit 5 mg trans-N-(4-Chlorbenzoyl)-N-methyl-4-[4-(3-hydroxypropyl)-methylaminomethylphenyl]cyclohexylamin

Zusammensetzung:

1 Zäpfchen enthält:

| | |
|---|---|
| Wirkstoff | 5,0 mg |
| Zäpfchenmasse (z.B. Witepsol W 45(R)) | 1 695,0 mg |
| | 1 700,0 mg |

Herstellungsverfahren:

Die feinpulverisierte Wirksubstanz wird in der geschmolzenen und auf 40 °C abgekühlten Zäpfchenmasse suspendiert. Man gießt die Masse bei 37 °C in leicht vorgekühlte Zäpfchenformen aus.

Zäpfchengewicht 1,7 g

Beispiel IV

Kapseln mit 5 mg trans-N-(4-Chlorbenzoyl)-N-methyl-4-[4-(3-hydroxypropyl)methylaminomethylphenyl]-cyclohexylamin

Zusammensetzung:

1 Kapsel enthält:

| | |
|---|---|
| Wirksubstanz | 5,0 mg |
| Lactose | 82,0 mg |
| Stärke | 82,0 mg |
| Magnesiumstearat | 1,0 mg |
| | 170,0 mg |

Herstellungsverfahren:

Die Pulvermischung wird intensiv gemischt und auf einer Kapselabfüllmaschine in Hartgelatine-Steckkapseln der Größe 3 abgefüllt, wobei das Endgewicht laufend überprüft wird.

Beispiel V

Tabletten mit 5 mg trans-4-[4-(3-Hydroxypropyl)methylaminomethylphenyl]-N-methyl-N-(4-trifluormethylbenzoyl)cyclohexylamin

Zusammensetzung:

1 Tablette enthält:

| | |
|---|---|
| Wirkstoff | 5,0 mg |
| Milchzucker | 148,0 mg |
| Kartoffelstärke | 65,0 mg |
| Magnesiumstearat | 2,0 mg |
| | 220,0 mg |

Herstellungsverfahren:

Aus Kartoffelstärke wird durch Erwärmen ein 10%iger Schleim hergestellt. Die Wirksubstanz, Milchzucker und die restliche Kartoffelstärke werden gemischt und mit obigem Schleim durch ein Sieb der Maschenweite 1,5 mm granuliert. Das Granulat wird bei 45°C getrocknet, nochmals durch obiges Sieb gerieben, mit Magnesiumstearat vermischt und zu Tabletten verpreßt.

| | |
|---|---|
| Tablettengewicht: | 220 mg |
| Stempel: | 9 mm |

Beispiel VI

Creme für die topische Verabreichung mit 1 g trans-N-(4-Chlorbenzoyl)-N-methyl-4-[4-(3-hydroxypropyl)-methylaminomethylphenyl]cyclohexylamin

Eine Formulierung für die topische Verabreichung der Verbindungen der Formel I kann folgende Zusammensetzung aufweisen

| | | |
|---|---|---|
| 1. | Wirkstoff | 1,0 g |
| 2. | Stearylalkohol | 4,0 g |
| 3. | Cetylalkohol | 4,0 g |
| 4. | Mineralöl | 3,0 g |
| 5. | Polysorbat 60 | 4,5 g |
| 6. | Sorbitanstearat | 4,5 g |
| 7. | Propylenglycol | 10,0 g |
| 8. | Methylparaben | 0,18 g |
| 9. | Propylparaben | 0,02 g |
| 10. | Wasser | q.s. ad 100,00 g |

Die Bestandteile 2-6 werden auf 80 °C erwärmt bis alles geschmolzen ist. Danach wird Bestandteil 1 in der öligen Phase gelöst. Bestandteil 7 und 10 werden auf 90 °C erwärmt und die Bestandteile 8 und 9 werden in der so erhaltenen wässrigen Phase gelöst. Danach wird die wässrige Phase zur Ölphase gegeben und rasch gerührt, so daß eine Emulsion erhalten wird. Danach läßt man langsam auf 50 °C abkühlen um die Emulsion zu verfestigen. Unter weiterem Rühren wird das Präparat auf Raumtemperatur abgekühlt.

Das folgende Beispiel beschreibt die Herstellung eines Futtermittels für Legehennen:

Beispiel VII

Futtermittel für Legehennen, enthaltend als Wirkstoff trans-N-(4-Chlorbenzoyl)-N-methyl-4-[4-(3-hydroxypropyl)methylaminomethylphenyl]cyclohexylamin

| | |
|---|---|
| Mais | 633 g/kg |
| Sojabohnenmehl | 260 g/kg |
| Fleischmehl | 40 g/kg |
| Futterfett | 25 g/kg |
| Sojaöl | 17 g/kg |
| Bicalciumphosphat | 12 g/kg |
| Calciumcarbonat | 6 g/kg |
| Vitamin-Mineralstoffmischung | 5 g/kg |
| Wirkstoff | 2 g/kg |

Diese Komponenten in den angegebenen Mengen ergeben nach sorgfältigem Mischen 1 kg Futter.

**Patentansprüche**

**1.** N,N-Disubstituierte Arylcycloalkylamine der allgemeinen Formel I,

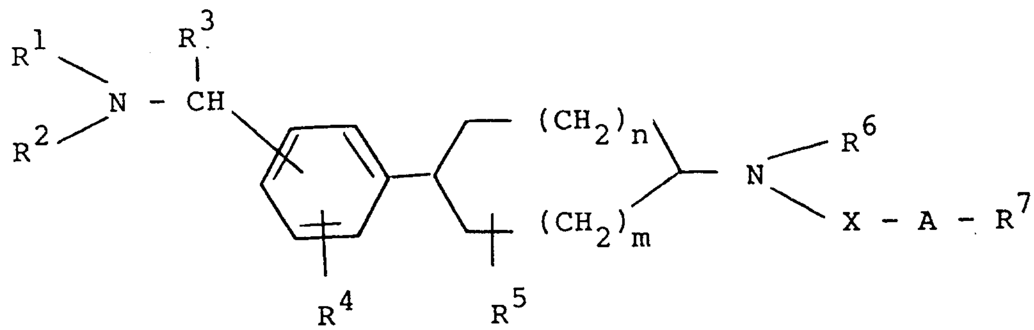

(I)

in der
n die Zahlen 1 oder 2,
m die Zahlen 0 oder 1,
A eine Einfachbindung, eine geradkettige oder verzweigte Alkylengruppe mit 1 bis 17 Kohlenstoffatomen, eine Alkenylengruppe mit 2 bis 17 Kohlenstoffatomen oder eine Alkinylengruppe mit 2 bis 4 Kohlenstoffatomen,
X eine Carbonyl- oder Sulfonylgruppe,
$R^1$ eine geradkettige oder verzweigte Alkylgruppe mit 1 bis 5 Kohlenstoffatomen,
$R^2$ eine geradkettige oder verzweigte Alkylgruppe mit 1 bis 5 Kohlenstoffatomen, die durch eine oder zwei Hydroxygruppen, eine Alkoxy- oder durch eine Alkylcarbonyloxygruppe mit 1 bis 5 Kohlenstoffatomen im Alkylteil, wobei der Alkylteil geradkettig oder verzweigt sein kann, durch eine Alkoxycarbonyloxygruppe, wobei die vorstehend erwähnten Substituenten nicht in Position 1 der Alkylgruppe gebunden sein können und zwei dieser Reste nicht an dasselbe Kohlenstoffatom gebunden sein können, sowie durch eine Aminocarbonyl-, Alkylaminocarbonyl-, Dialkylaminocarbonyl-, Cyano- oder Alkylcarbonylgruppe substituiert sein kann,
$R^3$, $R^4$ und $R^5$, die gleich oder verschieden sein können, jeweils ein Wasserstoffatom oder eine Alkylgruppe,
$R^6$ ein Wasserstoffatom, eine geradkettige oder verzweigte Alkylgruppe mit 1 bis 6 Kohlenstoffatomen, eine Cycloalkylgruppe mit 3 bis 6 Kohlenstoffatomen, eine Allyl-, Propargyloder eine gegebenenfalls durch ein Halogenatom substituierte Benzylgruppe,
$R^7$ ein Wasserstoffatom, eine Cycloalkylgruppe mit 3 bis 6 Kohlenstoffatomen, eine gegebenenfalls durch eine Alkylgruppe, durch ein oder zwei Halogenatome oder eine Trifluormethylgruppe substituierte Phenylgruppe, eine Naphthyl-, Tetrahydronaphthyl- oder eine gegebenenfalls durch ein Halogenatom oder eine Alkylgruppe substituierte Thienylgruppe bedeuten,
wobei A keine Einfachbindung sein kann, wenn X die Sulfonylgruppe und $R^7$ ein Wasserstoffatom bedeuten, und
wobei, sofern nichts anderes erwähnt wurde, die vorstehend erwähnten Alkyl- und Alkoxyteile jeweils 1 bis 3 Kohlenstoffatome enthalten können und die vorstehend erwähnten Halogenatome jeweils ein Fluor-, Chlor- oder Bromatom bedeuten können,
deren Enantiomere, Diastereomere und deren Salze.

**2.** N,N-Disubstituierte Arylcycloalkylamine der allgemeinen Formel I gemäß Anspruch 1,
in der
n die Zahl 1,
m die Zahl 1,
A eine Einfachbindung, eine geradkettige oder verzweigte Alkylengruppe mit 1 bis 17 Kohlenstoffatomen oder eine Alkenylengruppe mit 2 bis 4 Kohlenstoffatomen,
X eine Carbonyl- oder Sulfonylgruppe,
$R^1$ eine geradkettige oder verzweigte Alkylgruppe mit 1 bis 5 Kohlenstoffatomen,
$R^2$ eine geradkettige oder verzweigte Alkylgruppe mit 1 bis 5 Kohlenstoffatomen, die durch eine oder zwei Hydroxygruppen, eine Alkoxy- oder durch eine Alkylcarbonyloxygruppe mit 1 bis 5 Kohlenstoffatomen im Alkylteil, wobei der Alkylteil geradkettig oder verzweigt sein kann, durch eine Alkoxycarbonyloxygruppe, wobei die vorstehend erwähnten Substituenten nicht in Position 1 der Alkylgruppe gebunden sein können und zwei dieser Reste nicht an dasselbe Kohlenstoffatom gebunden sein können, sowie durch eine Aminocarbonyl-, Cyano- oder Alkylcarbonylgruppe substituiert sein kann,

$R^3$ ein Wasserstoffatom oder eine Alkylgruppe,
$R^4$ und $R^5$, die gleich oder verschieden sein können, jeweils ein Wasserstoffatom oder eine Methylgruppe,
$R^6$ eine geradkettige oder verzweigte Alkylgruppe mit 1 bis 6 Kohlenstoffatomen, eine Cycloalkylgruppe mit 3 bis 6 Kohlenstoffatomen, eine Allyl-, Propargyl- oder eine gegebenenfalls durch ein Halogenatom substituierte Benzylgruppe,
$R^7$ ein Wasserstoffatom, eine Cycloalkylgruppe mit 3 bis 6 Kohlenstoffatomen oder eine gegebenenfalls durch 1 oder 2 Halogenatome oder eine Trifluormethylgruppe substituierte Phenylgruppe bedeuten,
wobei A keine Einfachbindung sein kann, wenn X die Sulfonylgruppe und $R^7$ ein Wasserstoffatom bedeuten, und
wobei, sofern nichts anderes erwähnt wurde, die vorstehend erwähnten Alkyl- und Alkoxyteile jeweils 1 bis 3 Kohlenstoffatome enthalten können und die vorstehend erwähnten Halogenatome jeweils ein Fluor-, Chlor- oder Bromatom bedeuten können,
deren Enantiomere, Diastereomere und deren Salze.

**3.** N,N-Disubstituierte Arylcycloalkylamine der allgemeinen Formel I gemäß Anspruch 1,
in der
n die Zahl 1,
m die Zahl 1,
A eine Einfachbindung, eine geradkettige oder verzweigte Alkylengruppe mit 1 bis 17 Kohlenstoffatomen oder eine 2-Propenylengruppe,
X eine Carbonyl- oder Sulfonylgruppe,
$R^1$ eine geradkettige oder verzweigte Alkylgruppe mit 1 bis 5 Kohlenstoffatomen,
$R^2$ eine geradkettige oder verzweigte Alkylgruppe mit 1 bis 5 Kohlenstoffatomen, die durch eine oder zwei Hydroxygruppen, eine Alkoxy- oder durch eine Alkylcarbonyloxygruppe mit 1 bis 4 Kohlenstoffatomen im Alkylteil, wobei der Alkylteil geradkettig oder verzweigt sein kann, durch eine Alkoxycarbonyloxygruppe, wobei die vorstehend erwähnten Substituenten nicht in Position 1 der Alkylgruppe gebunden sein können und zwei dieser Reste nicht an dasselbe Kohlenstoffatom gebunden sein können, sowie durch eine Aminocarbonyl-, Cyano- oder Alkylcarbonylgruppe substituiert sein kann,
$R^3$ ein Wasserstoffatom oder eine Methylgruppe,
$R^4$ und $R^5$ jeweils ein Wasserstoffatom,
$R^6$ eine geradkettige oder verzweigte Alkylgruppe mit 1 bis 3 Kohlenstoffatomen, eine Cyclopropylgruppe, eine Allyl-, Propargyl- oder eine gegebenenfalls durch ein Fluoratom substituierte Benzylgruppe,
$R^7$ ein Wasserstoffatom, eine Cyclohexylgruppe, eine gegebenenfalls in 4-Stellung durch ein Chloratom oder eine Trifluormethylgruppe substituierte Phenylgruppe, die 3,4-Dichlorphenylgruppe, die 4-Chlor-3-methylphenylgruppe, die 5-Methyl-2-thienylgruppe, die 5-Chlor-2-thienylgruppe oder die 1,2,3,4-Tetrahydronaphthylgruppe bedeuten,
wobei A keine Einfachbindung sein kann, wenn X die Sulfonylgruppe und $R^7$ ein Wasserstoffatom bedeuten, und
wobei, sofern nichts anderes erwähnt wurde, die vorstehend erwähnten Alkyl- und Alkoxyteile jeweils 1 bis 3 Kohlenstoffatome enthalten können,
deren Enantiomere, Diastereomere und deren Salze.

**4.** Folgende Verbindungen der allgemeinen Formel I gemäß Anspruch 1:
(1) trans-N-(4-Chlorbenzolsulfonyl)-N-methyl-4-[4-(2-methoxyethyl)methylaminomethylphenyl]-cyclohexylamin
(2) trans-4-(4-Aminocarbonylmethyl-methylaminomethylphenyl)-N-(4-chlorbenzolsulfonyl)-N-methylcyclohexylamin
(3) trans-4-(4-Aminocarbonylmethyl-methylaminomethylphenyl)-N-(4-chlorbenzoyl)-N-methylcyclohexylamin
(4) trans-N-(4-Chlorbenzoyl)-N-methyl-4-[4-(3-hydroxypropyl)methylaminomethylphenyl]-cyclohexylamin
(5) trans-4-[4-(3-Acetoxypropyl)methylaminomethylphenyl]-N-(4-chlorbenzoyl)-N-methylcyclohexylamin
(6) trans-N-(4-Chlorbenzoyl)-N-methyl-4-[4-(3-cyanopropyl)methylaminomethylphenyl]cyclohexylamin
(7) trans-4-[4-(1-Aminocarbonylethyl)methylaminomethylphenyl]-N-(4-chlorbenzoyl)-N-methylcyclohexylamin

(8) trans-N-(4-Chlorbenzoyl)-N-methyl-4-[4-(R-2,3-dihydroxy-2-methylpropyl)-methylaminomethylphenyl]cyclohexylamin
(9) trans-N-(4-Chlorbenzoyl)-N-methyl-4-[4-(2-hydroxyethyl)isopropylaminomethylphenyl]-cyclohexylamin
(10) Cis-N-(4-Chlorbenzoyl)-N-methyl-4-[4-(3-hydroxypropyl)methylaminomethylphenyl]-cyclohexylamin
(11) trans-4-[4-(3-Hydroxypropyl)methylaminomethylphenyl]-N-methyl-N-(4-trifluormethylbenzoyl)-cyclohexylamin
(12) trans-N-Cyclohexylcarbonyl-N-methyl-4-[4-(3-hydroxypropyl)methylaminomethylphenyl]-cyclohexylamin
(13) trans-N-(4-Chlor-3-methylbenzoyl)-N-methyl-4-[4-(3-hydroxypropyl)methylaminomethylphenyl]-cyclohexylamin
(14) trans-N-(3,4-Dichlorphenylacetyl)-N-methyl-4-[4-(3-hydroxypropyl)methylaminomethylphenyl]-cyclohexylamin
(15) trans-4-[4-(3-Hydroxypropyl)methylaminomethylphenyl]-N-methyl-N-(1,2,3,4-tetrahydronaphthalin-2-carbonyl)cyclohexylamin
(16) trans-N-(5-Chlorthienyl-2-carbonyl)-N-methyl-4-[4-(3-hydroxypropyl)methylaminomethylphenyl]-cyclohexylamin

deren Enantiomere, Diastereomere und deren Salze.

**5.** Physiologisch verträgliche Salze der Verbindungen nach mindestens einem der Ansprüche 1 bis 4 mit anorganischen oder organischen Säuren.

**6.** Arzneimittel, enthaltend eine Verbindung nach mindestens einem der Ansprüche 1 bis 4 oder ein physiologisch verträgliches Salz gemäß Anspruch 5 neben gegebenenfalls einem oder mehreren inerten Trägerstoffen und/oder Verdünnungsmitteln.

**7.** Verwendung einer Verbindung nach mindestens einem der Ansprüche 1 bis 5 zur Herstellung eines Arzneimittels zur Inhibition der Cholesterolbiosynthese, zur Behandlung oder Prophylaxe von Hyperlipidämien, zur Behandlung von Erkrankungen, die mit überhöhter Zellproliferation im Zusammenhang stehen, zur Prophylaxe und Behandlung von Gallensteinleiden oder zur Behandlung von Mykosen.

**8.** Verwendung einer Verbindung nach mindestens einem der Ansprüche 1 bis 5 zur Herstellung eines Futtermittels für Legehennen zur Erzeugung cholesterolarmer Eier.

**9.** Verfahren zur Herstellung eines Arzneimittels gemäß Anspruch 6, dadurch gekennzeichnet, daß auf nichtchemischem Wege eine Verbindung nach mindestens einem der Ansprüche 1 bis 5 in einen oder mehrere inerte Trägerstoffe und/oder Verdünnungsmittel eingearbeitet wird.

**10.** Verfahren zur Herstellung der Verbindungen nach mindestens einem der Ansprüche 1 bis 5, dadurch gekennzeichnet, daß

a) eine Verbindungen der allgemeinen Formel II,

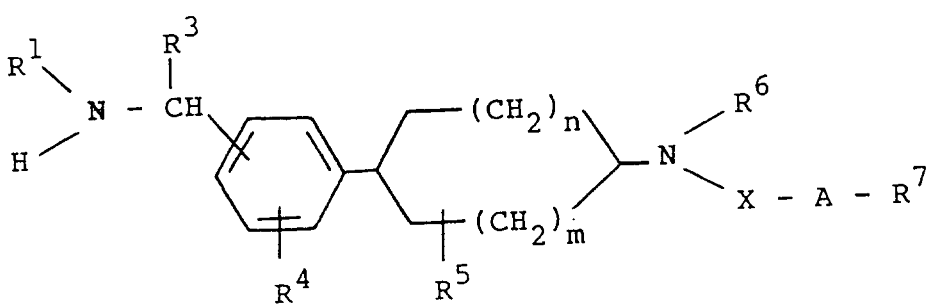

(II)

in der
n, m, A, X, $R^1$ und $R^3$ bis $R^7$ die in den Ansprüchen 1 bis 4 erwähnten Bedeutungen besitzen, mit einer Verbindung der allgemeinen Formel III,

$R^2$ - Y (III)

in der
$R^2$ die in den Ansprüchen 1 bis 4 erwähnten Bedeutungen besitzt und Y eine reaktive Austrittsgruppe bedeutet, umgesetzt wird oder
b) zur Herstellung von Verbindungen der allgemeinen Formel I, in der $R^2$ eine Gruppe der allgemeinen Formel IV,

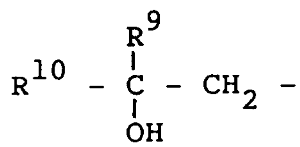

(IV)

in der
$R^9$ ein Wasserstoffatom oder eine Methylgruppe und $R^{10}$ eine Methyl- oder Hydroxymethylgruppe bedeutet, eine Verbindung der allgemeinen Formel II, in der n, m, A, X, $R^1$ und $R^3$ bis $R^7$ die in den Ansprüchen 1 bis 4 erwähnten Bedeutungen besitzen, mit einem Epoxid der allgemeinen Formel V,

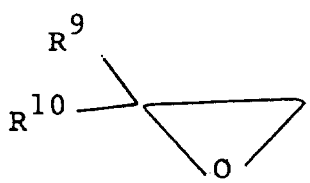

(V)

in der
$R^9$ ein Wasserstoffatom oder eine Methylgruppe und $R^{10}$ eine Methyl- oder Hydroxymethylgruppe bedeutet, umgesetzt wird oder
c) eine Verbindung der allgemeinen Formel VI,

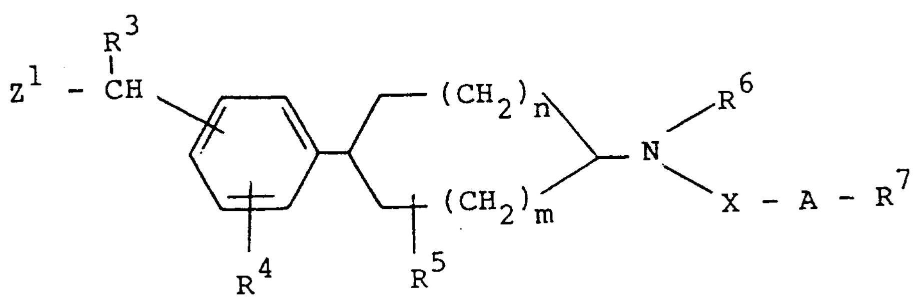

(VI)

in der
n, m, A, X und $R^3$ bis $R^7$ die in den Ansprüchen 1 bis 4 erwähnten Bedeutungen besitzen und $Z^1$ eine reaktive Austrittsgruppe bedeutet, mit einem Amin der allgemeinen Formel VII,

$R^1R^2NH$ (VII)

in der
$R^1$ und $R^2$ die in den Ansprüchen 1 bis 4 erwähnten Bedeutungen besitzen, umgesetzt wird oder
d) eine Verbindung der allgemeinen Formel VIII,

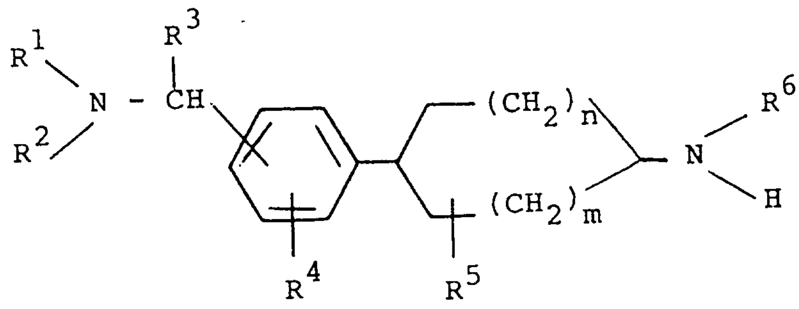

(VIII)

in der

n, m und $R^1$ bis $R^6$ die in den Ansprüchen 1 bis 4 erwähnten Bedeutungen besitzen, mit einem Alkylierungsmittel der allgemeinen Formel IX,

$R^7$ - A - X - $Z^2$ ,(IX)

in der

A, X und $R^7$ die in den Ansprüchen 1 bis 4 erwähnten Bedeutungen besitzen und $Z^2$ eine reaktive Austrittsgruppe bedeutet, umgesetzt wird und

gewünschtenfalls eine Verbindung der allgemeinen Formel I, in der $R^2$ eine durch eine Hydroxygruppe substituierte Alkylgruppe darstellt, durch Umsetzung mit einem geeigneten Acylierungsmittel in eine Verbindung der allgemeinen Formel I, in der $R^2$ eine durch eine Alkylcarbonyloxy- oder Alkoxycarbonyloxygruppe substituierte Alkylgruppe darstellt, übergeführt wird oder

gewünschtenfalls eine Verbindung der allgemeinen Formel I, in der $R^2$ eine durch eine Hydroxygruppe substituierte Alkylgruppe darstellt, mittels Oxidation in eine Verbindung der allgemeinen Formel I, in der $R^2$ eine Alkylcarbonylmethylgruppe darstellt, übergeführt wird oder

gegebenenfalls eine oder zwei in einer Verbindung der allgemeinen Formel VIII vorhandene Hydroxygruppen bei der Durchführung der Umsetzung d) durch Verwendung von zwei oder drei Äquivalenten einer Verbindung der allgemeinen Formel IX in die entsprechenden Estergruppen übergeführt werden und die Esteigruppen anschließend wieder verseift werden oder

eine so erhaltene Verbindung der allgemeinen Formel I in ihr Salz mit einer anorganischen oder organischen Säure übergeführt wird.

Europäisches Patentamt

# EUROPÄISCHER RECHERCHENBERICHT

Nummer der Anmeldung
EP 93 11 8595

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| A | EP-A-0 464 465 (F. HOFFMANN-LAROCHE AG)<br>--- | | C07C233/79<br>C07C311/20<br>C07C237/06<br>C07C255/24<br>A61K31/16<br>A61K31/18<br>C07C233/41<br>C07C311/07<br>C07C233/62<br>C07D333/38 |
| A | US-A-3 979 444 (D. LEDNICER)<br>----- | | |

| RECHERCHIERTE SACHGEBIETE (Int.Cl.5) |
|---|
| C07C<br>A61K |

Der vorliegende Recherchenbericht wurde für alle Patentansprüche erstellt

| Recherchenort | Abschlußdatum der Recherche | Prüfer |
|---|---|---|
| DEN HAAG | 21. März 1994 | Pauwels, G |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument